# EUROPEAN PATENT APPLICATION

(11) **EP 3 978 028 A1**
(43) Date of publication of application: **06.04.2022**
(21) Application number: 21182899.1
(22) Date of filing: 30.06.2021
(51) Int. Cl.: A61K 47/69, A61K 31/69, A61K 33/22, A61K 47/42, A61K 47/68, A61P 21/00, A61P 35/00, C07K 5/09, C07K 5/103, C07K 5/11, C07K 7/06, C07K 7/08, C07K 14/78, C07K 17/06, C07K 17/14

(54) **NEW COMBINATIONS OF BORON COMPOUNDS AND ADJUVANTS FOR THE TREATMENT OF PATHOPHYSIOLOGICAL CONDITIONS AND MUSCLE REGENERATION**

(30) Priority: 02.10.2020 EP 20382876
(71) Applicant: Consorcio Centro de Investigación Biomédica en Red M.P., 28029 Madrid (ES)
(72) Inventor: RICO TORTOSA, Patricia, 46011 Valencia (ES); RODRÍGUEZ ROMANO, Ana, 46011 Valencia (ES)
(74) Representative: Isern Patentes y Marcas S.L.

(57) **Abstract**

The invention relates to a new combinations of boron compounds and adjuvants and compositions thereof, suitable for use in a method to induce myotube formation and suppress cell mortality in a mammal in need thereof. Furthermore, it is related to compositions comprising boron compounds, adjuvants and support platforms, which enhance the effect of synergistic activation of the boron cell membrane transporter (NaBC1) and adhesion receptors in cells. The invention also refers to the aforementioned combinations and compositions for the treatment of pathophysiological condition, which affects skeletal muscle, such as dystrophy. Therefore, the present invention can be included in the field of medical chemistry or pharmacology.

SEQUENCE LISTING

<110>  Centro de Investigación Biomédica en Red (CIBER)

<120>  New combinations of boron compounds and adjuvants for the
       treatment of pathophysiological conditions and muscle
       regeneration

<130>  BBN2103

<150>  EP20382876
<151>  2020-10-02

<160>  5

<170>  PatentIn version 3.5

<210>  1
<211>  4
<212>  PRT
<213>  Artificial Sequence

<220>
<223>  RGD peptide

<400>  1

Arg Gly Asp Arg
1


<210>  2
<211>  5
<212>  PRT
<213>  Artificial Sequence

<220>
<223>  IKVAV laminin domain

<400>  2

Ile Lys Val Ala Val
1               5


<210>  3
<211>  7
<212>  PRT
<213>  Artificial Sequence

<220>
<223>  RGD peptide

<400>  3

Gly Arg Gly Asp Ser Pro Cys
1               5


<210>  4
<211>  19
<212>  PRT
<213>  Artificial Sequence

<220>
<223>  IKVAV laminin domain

<400>  4

Cys Ser Arg Ala Arg Lys Gln Ala Ala Ser Ile Lys Val Ala Val Ser
1               5                   10                  15

Ala Asp Arg

<210>  5
<211>  16
<212>  PRT
<213>  Artificial Sequence

<220>
<223>  VPM peptide

<400>  5

Gly Cys Arg Asp Val Pro Met Ser Met Arg Gly Gly Asp Arg Cys Gly
1               5                   10                  15

## Description

### Field of the invention

The invention relates to a new combinations of boron compounds and adjuvants for use in a method to induce myotube formation and suppress cell mortality in a mammal in need thereof and compositions thereof.

Furthermore, it related to compositions comprising boron compounds, adjuvants and a support platform (also referred as supports) suitable for simultaneous solid-phase presentation of boron and cell adhesion domains. In particular, the material platforms can present only boron for targeting the borate transporter NaBC1, only cell adhesion domains for targeting integrins, and also others simultaneously presenting boron and cell adhesion domains for simultaneous targeting of NaBC1 transporter and integrins.

The invention also refers to the aforementioned combinations and compositions for the treatment of pathophysiological condition, which affects skeletal muscle, such as dystrophy. Therefore, the present invention can be included in the field of medical chemistry or pharmacology.

### Background of the Invention

Boron is an essential metalloid that plays a key role in the metabolisms of plants and animals. It has been seen that boron is involved in bone mineralization. Among other uses it has been employed in synthesis chemistry and is has been recently exploited in medical-chemistry. Actually, little is known about homeostasis and boron function in animal cells; however in the latter, boron has been reported to be involved in myogenic differentiation in mice (Tissue Eng Part A. 201, Nov; 21 (21-22) :2662-7) and muscular dystrophy.

There are different types of dystrophies, for instance, Duchenne muscular dystrophy (DMD) is a progressive and lethal disease, caused by mutations associated with the X chromosome of the gene that encodes dystrophin. Lack of dystrophin leads to weakness, degeneration and consequent fibrosis in the skeletal and cardiac muscles. Currently, there is no cure for DMD patients. Some of pre-clinical and clinical approaches in development, include exon omission, genetic editing through viral vectors, and stem cell transplants (Stem Gel/ Reviews and Reports, Vol. 14, 2018).

All of these approaches will take some time for them to become available, and even when approved they may only be suitable for one population selected patients and/or have the ability to improve symptoms but not completely relieve them. Therefore, therapies are needed that, either independently or in combination with other treatments, slow or ease the disease progression. Myotonic dystrophy (DM) is a dominantly inherited neuromuscular disorder without currently cure or effective treatment, wherein some therapeutic advances have been developed, however, without ample success (Drug Discovery Today. 2017; 22: 1740-1748; Drug Discovery Today. 2018; 00: 1-10). Some of these therapies have employed active ingredients known for other therapies, for instance mexiletine, an antiarrhythmic that acts on sodium channels, as "off-label" treatments to treat myotonia in DM and non-dystrophy myotonia. Preliminary studies have suggested that recombinant human IGF1 (rhIGF1) can improve muscle strength and function in adult patients with DM1, although this therapy has significant limitations. Other important work to treat DM was focused on designing anti-sense oligonucleotides to neutralize defective mRNA in these patients *(*Drug Discovery All and. 2018; 00: 1-1 0). However, these approaches to DM treatment are used as supportive aids and do not slow or stop disease progression.

A latter publication, ES 2 749 465 A1 is based on the soluble combination of some basic boron salts such as borax, experimental results in flies and its potential use in the treatment of muscular dystrophies. However, the aforementioned combinations allow the use of lower dosages of the borax when combined with fibronectin, these are may still need to be further reduced if boron compounds wish to be used in pharmaceutical compositions for the treatment of muscular diseases or other medical treatments.

Therefore, there is a need to minimize the physiological effect of defective mRNA to provide a quality of life for patients with this rare disease as well as minimizing and reducing the dosages of the suitable boron compounds to non-toxic dosages, to ensure effectivity while reducing or eliminating side effects.

### Brief description of the invention

The first aspect of the invention relates to a combination of a boron compound and an adjuvant for use in a method to induce myotube formation and suppress cell mortality in a mammal in need thereof, wherein the adjuvant comprises fibronectin, cell adhesion peptide domains or mixtures thereof and provided that when the adjuvant is fibronectin, the boron compound is not borax.

In the combination of the first aspect, when the adjuvant comprises fibronectin, the ratio of boron compound to the adjuvant is from 1.10⁻¹ to 1.10⁻⁶, preferably from 1.10⁻² to 1.10⁻⁶ by weight and/or wherein the adjuvant comprises cell adhesion peptide domains, the ratio of boron compound to the adjuvant is from 10 to 10⁻⁶, preferably from 5 to 10⁻⁶ by weight. The aforementioned ranges allow the use of ultra-low doses of boron compounds, while improving the safety and ensuring efficacy. Ranges lower than the ones above will provide lower efficacy. Ranges higher than the ones above may increase toxicity and do not ensure efficacy.

The combination of the first aspect allows the use of ultra-low doses of boron compounds, bellow toxic concentrations, improving the safety of these compounds while ensuring efficacy. Thus, the combination of the first aspect, improve myotube formation and myotube diameter as well as it suppressed cell mortality even when using high concentrations of the boron compound. Consequently, these combinations are suitable for biomedical applications, as they can enhance the effect of synergistic activation of the boron transporter NaBC1 and adhesion receptors in cells.

In a preferred embodiment of the invention, the mammal is a human in need thereof.

The second aspect of the invention relates to a pharmaceutical composition comprising the combinations of the first aspect, thus comprising at least;
- A boron compound;
- at least an adjuvant which comprises fibronectin, cell adhesion peptide domains or mixtures thereof; provided that when the adjuvant is fibronectin, the boron compound is not borax; and
- optionally at least a pharmaceutical acceptable excipient.
- Optionally, when the adjuvant comprises fibronectin, the ratio of boron compound to the adjuvant is from 1.10⁻¹ to 1.10⁻⁶, preferably from 1.10⁻² to 1.10⁻⁶ by weight and/or wherein the adjuvant comprises cell adhesion peptide domains, the ratio of boron compound to the adjuvant is from 10 to 1.10⁻⁶, preferably from 5 to 1.10⁻⁶ by weight.

The pharmaceutical compositions of the second aspect allow the use of ultra-low doses of boron compounds, bellow toxic concentrations, improving the safety of these compounds while ensuring efficacy. The aforementioned ranges allow the use of ultra-low doses of boron compounds, while improving the safety and ensuring efficacy. Ranges lower than the ones above will provide lower efficacy. Ranges higher than the ones above may increase toxicity and do not ensure efficacy.

Thus, the composition of the second aspect, improves myotube formation and myotube diameter as well as it suppresses cell mortality even when using high concentrations of the boron compound. Thus, these compositions are suitable to be used for the treatment of pathophysiological conditions, which affect skeletal muscle. Preferably said pathophysiological condition is a dystrophy, even more preferably wherein the dystrophy is selected from the list consisting of myotonic dystrophy type 1, myotonic dystrophy type 2 and Duchenne muscular dystrophy. They are also suitable to be used in a method to improve muscle regeneration.

Likewise, the combinations of the first aspect as well as the compositions of the second and third aspects are suitable to be used as regenerative therapeutic treatments selected form the list consisting of, muscle repair, muscle regeneration, as a consequence of an injury due to sport lesions, injuries in general or muscle defects naturally generated by aging.

The third aspect of the invention relates to a pharmaceutical composition comprising;
- A boron compound;
- at least an adjuvant which comprises fibronectin or cell adhesion peptide domains;
- a support which comprises a substrate selected from the list consisting of hydrogel matrices, glassware and silica nanoparticles; and
- Optionally at least a pharmaceutical acceptable excipient.

The presence of the above support enhances the effect of synergistic activation of the boron cell membrane transporter (NaBC1) and adhesion receptors in cells, in particular as suitable matrices for use in the medical and pharmaceutical field. More particularly, these compositions are suitable matrices for use in regenerative therapeutic treatments and represent a suitable system for use in a pharmaceutical composition comprising the boron compounds of the invention. Preferably, regenerative therapeutic treatments are selected form muscle repair, muscle regeneration, as a consequence of an injury due to sport lesions, injuries in general or muscle defects naturally generated by aging.

The compositions of the third aspect have the advantage that they allow the use of ultra-low doses of boron compounds, due to the simultaneous presence of the boron compounds and the cell adhesion domains, that generates a synergic and collaborative effect of cell membrane receptors stimulating intracellular signalling, thus allowing the applicability of this materials/compositions and its combination with boron compounds in the biomedical field. These compositions reduce significantly the toxicity of the boron compounds when used in mammals, while ensuring a good efficacy. Thus, these pharmaceutical compositions are suitable to be used in the treatment of pathophysiological condition that affect skeletal muscle, preferably said condition is a dystrophy, even more preferably wherein the dystrophy is selected from the list consisting of myotonic dystrophy type 1, myotonic dystrophy type 2 and Duchenne muscular dystrophy.

The fourth aspect of the present invention relates to the combination of the first aspect and the compositions of the second and third aspects for use in a method to improve muscle regeneration.

The fifth aspect of the present invention relates to the combination of the first aspect, the composition of the second or the third aspect for use in a method of treatment or therapy of pathophysiological condition which affects skeletal muscle, preferably said condition is a dystrophy, preferably, the dystrophy is selected from the list consisting of myotonic dystrophy type 1, myotonic dystrophy type 2 and Duchenne muscular dystrophy.

The sixth aspect of the present invention relates to the compositions of the second or third aspects for use in a method of treatment or therapy of pathophysiological condition which affects skeletal muscle, wherein said method comprises administering the boron compound to said mammal in an amount effective for decreasing muscle atrophy and/or in amount effective to increase muscle growth in said mammal. Preferably, the boron compound is bortezomib and is administered to a human in an effective dose from 0.1 to 1 mg once or twice weekly, preferably, from 0.1 to 0.5 mg once or twice weekly.

The seventh aspect of the present invention relates to the combination of the first aspect and the composition of the second aspect for use as regenerative therapeutic treatments selected form the list consisting of, muscle repair, muscle regeneration, as a consequence of an injury due to sport lesions, injuries in general or muscle defects naturally generated by aging.

The eighth aspect of the present invention relates to the use of the composition of the second or the third aspect as cell culture substrates for muscle cell biopsies from patients, preferably, for muscle cell dystrophic biopsies or an immortalised dystrophic cell lines.

The ninth aspect of the present invention is related to a method to prepare the composition of the second aspect, comprising at least the steps of:
- Providing the boron compound;
- Mixing the boron compound with the adjuvant in a ratio of boron compound to the fibronectin from 1.10⁻¹ to 1.10⁻⁶, preferably from 1.10⁻² to 1.10⁻⁶ by weight and/or wherein the adjuvant comprises cell adhesion peptide domains, the ratio of boron compound to the adjuvant is from 10 to 1.10⁻⁶, preferably from 5 to 1.10⁻⁶ by weight; and
- Optionally adding a pharmaceutical acceptable excipient.

The tenth aspect of the present invention relates to a method to prepare the composition of the second aspect comprising the support, the method comprises at least the steps of:
a) Preparing the functionalised support by chemically modifying the surface of the support by silanization and further reacting with a compound having a terminal alkyne group;
b) Incorporating the boron compound in the functionalised support of the previous step by performing a coupling reaction on the product obtained in the previous step using a Pd-mediated cross-coupling agent to incorporate the boron compound into the linker, wherein the linker chain comprises a terminal azide group;
c) azide-alkyne click coupling between the modified surface from step (a) and the boron-containing linker from step (b); and
d) performing a maleimide-thiol coupling between the support obtained in step (c) and adjuvant, wherein the adjuvant contains the appropriate cysteine-terminated peptides containing specific integrin adhesion sequences, preferably wherein the adjuvant is Fibronectin or a Cell adhesion domain peptide, which comprises or consists of a sequence selected from a list consisting of the SEQ ID NO: 1, SEQ ID NO: 2, SEQ ID NO: 3 and SEQ ID NO: 4.

### BRIEF DESCRIPTION OF THE FIGURES

The above and other advantages and features will be understood from the following description of the embodiments and examples with reference to the figures. A series of figures have been included, the description of which is as follows:
Figure 1: Immunofluorescence images of myotube formation after 8 days of culture of human wild (WT) type cells without or with fibronectin and different concentrations of bortezomib, detected with sarcomeric α-actinin muscle marker.
Figure 2: Immunofluorescence images of myotube formation after 8 days of culture of human DMD cells without or with fibronectin and different concentrations of bortezomib, detected with sarcomeric α-actinin muscle marker.
Figure 3: Immunofluorescence images of myotube formation after 8 days of culture of human DM1 cells without or with fibronectin and different concentrations of bortezomib, detected with sarcomeric α-actinin muscle marker.

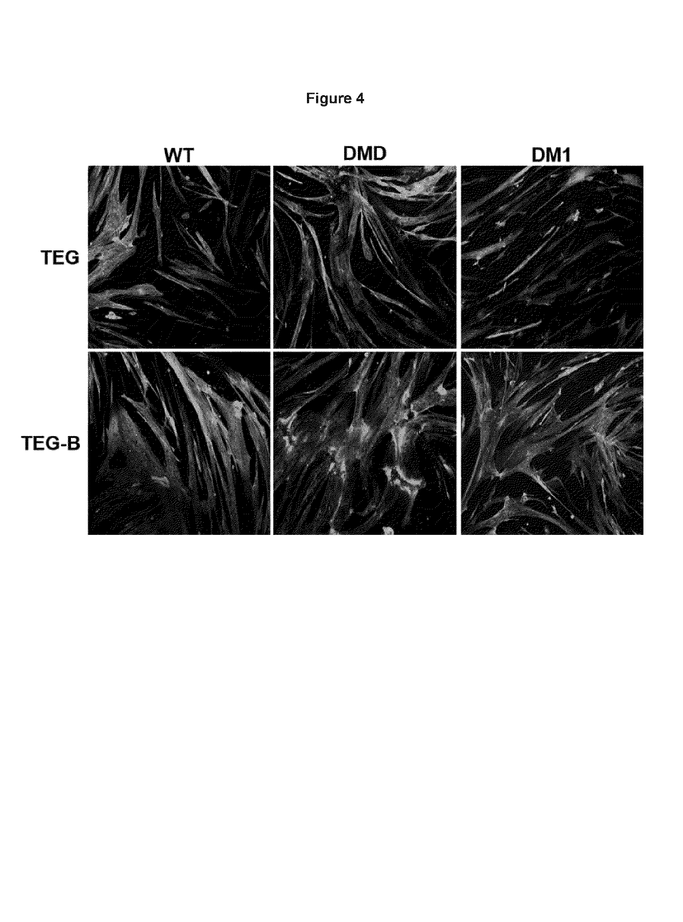

Figure 4: Immunofluorescence images of myotubes detected with sarcomeric α-actinin muscle marker after 6 days of culture in WT, DMD and DM1 type cells seeded onto FN-coated supports presenting tetraethylenglycol chains (TEG) chemically anchored in their surface or TEG-B covalently bound to boron compounds.

### Detailed description of the invention

The first aspect of the invention relates to a combination of a boron compound and an adjuvant for use in a method to induce myotube formation and suppress cell mortality in a mammal in need thereof, wherein the adjuvant comprises fibronectin, cell adhesion peptide domains or mixtures thereof and provided that when the adjuvant is fibronectin, the boron compound is not borax.

In the combination of the first aspect, when the adjuvant comprises fibronectin, the ratio of boron compound to the adjuvant is from 1.10⁻¹ to 1.10⁻⁶, preferably from 1.10⁻² to 1.10⁻⁶ by weight and/or wherein the adjuvant comprises cell adhesion peptide domains, the ratio of boron compound to the adjuvant is 10 to 1.10⁻⁶, preferably from 5 to 1.10⁻⁶ by weight. Preferably, when the adjuvant comprises fibronectin, the ratio of boron compound to the adjuvant is from 1.10⁻² to 1.10⁻⁵ by weight, more preferably from 1.10⁻³ to 1.10⁻⁵ and/or wherein the adjuvant comprises cell adhesion peptide domains, the ratio of boron compound to the adjuvant is from 5 to 1.10⁻⁶ by weight. The use of the adjuvant allows the reduction of the amount of the boron compound even to amounts that are not toxic.

In the context of the present invention, the term adjuvant refers to fibronectin or to cell adhesion peptide domains of the fibronectin molecule or mixtures thereof.

In the context of the present invention, the term fibronectin refers to a high-molecular weight (approximately 500 kDa measured by GPC) glycoprotein of the extracellular matrix that binds to-spanning receptor proteins called integrins. "The term "fibronectin", as used herein, refers to the protein with the amino acid sequence disclosed in the UniProtKB database, with accession number P02751 (FINC_HUMAN), version 16 October 2019. In a particular embodiment, it refers to the protein with the amino acid sequence of any of the isoforms disclosed in said version of the accession number of the UniProtKB database, preferably with the amino acid sequence of the canonical isoform, or isoform 15 referred therein. The term fibronectin extended to the functional equivalence of fibronectin, with a similarity of 70%, 75%, 80%, 85%, 90%, 95% or 100%.

Fibronectin exists as a protein dimer, consisting of two nearly identical polypeptide chains linked by a pair of C-terminal disulfide bonds. Each fibronectin subunit has a molecular weight of 230-250 kDa and contains three types of modules: type I, II, and III. All three modules are composed of two anti-parallel β-sheets resulting in a Beta-sandwich. Three regions of variable splicing occur along the length of the fibronectin promoter. One or both of the "extra" type III modules (EIIIA and EIIIB) may be present in cellular fibronectin, but they are never present in plasma fibronectin. A "variable" V-region exists between III₁₄₋₁₅ (the 14th and 15th type III module). The V-region structure is different from the type I, II, and III modules, and its presence and length may vary. The V-region contains the binding site for α4β1 integrins. It is present in most cellular fibronectin, but only one of the two subunits in a plasma fibronectin dimer contains a V-region sequence.

The modules are arranged into several functional and protein-binding domains along the length of a fibronectin monomer. There are four fibronectin-binding domains, allowing fibronectin to associate with other fibronectin molecules.

One of these fibronectin-binding domains, I₁₋₅, is referred to as the "assembly domain", and it is required for the initiation of fibronectin matrix assembly.

Modules III₉₋₁₀ correspond to the "cell-binding domain" of fibronectin. The RGD sequence (Arg-Gly-Asp) is located in III₁₀ and is the site of cell attachment via α5β1 and αVβ3 integrins on the cell surface. The "synergy site" is in III9 and has a role in modulating fibronectin's association with α5β1 integrins.

Fibronectin also contains domains for fibrin-binding (I₁₋₅, I₁₀₋₁₂), collagen-binding (I₆₋₉), fibulin-1-binding (III₁₃₋₁₄), heparin-binding and syndecan-binding (III₁₄).

In the case of the cell adhesion peptide domains of the fibronectin molecule, only certain biologically active domains of the fibronectin molecule can activate integrins. The cell adhesion peptides domains of the invention derive from proteins responsible for cell-to-cell adhesion or for cell to extracellular matrix adhesion. Suitable domains are selected from the cell adhesion peptide domain RGD (Arginine, Glycine, and Aspartate), LDV domain (Lysine, Aspartate, Valine) or other extracellular protein fibronectin domains and a cell adhesion peptide domain from the extracellular protein laminin Peptide domain from the IKVAV (Isoleucine, Lysine, Valine, Alanine and Valine).

In another preferred embodiment of the combination of the first aspect the boron compound is bortezomib.

In a preferred embodiment of the combination of the first aspect, the boron compound is bortezomib, the adjuvant is fibronectin and the ratio of bortezomib to adjuvant is from 10⁻⁶ to 10⁻³ by weight.

In another preferred embodiment of the combination of the first aspect, the boron compound is bortezomib, the adjuvant is cell adhesion peptide domains and the ratio of bortezomib to adjuvant is from 0.01 to 10⁻⁶ by weight.

In a preferred embodiment, the cell adhesion peptide domains comprise a sequence selected from a list consisting of: the SEQ ID NO: 1, SEQ ID NO: 2, SEQ ID NO: 3 and SEQ ID NO: 4. In a more preferred embodiment, cell adhesion peptide domain comprises cysteine-terminated peptides containing specific integrin adhesion sequences. More preferably, the cell adhesion peptide domain comprises a sequence selected from a list consisting of: the SEQ ID NO: 1 (RGD), SEQ ID NO: 2 (IKVAV), SEQ ID NO: 3 (GRGDSPC) and SEQ ID NO: 4 (CSRARKQAASIKVAVSADR).

The combinations of the first aspect are suitable to be used in a mammal, preferably in humans, in an amount effective for decreasing muscle atrophy or to increase muscle growth in said mammal. Preferably, wherein said mammal has a pathophysiological condition affecting skeletal muscle, preferably said condition is a dystrophy, even more preferably wherein the dystrophy is selected from the list consisting of myotonic dystrophy type 1, myotonic dystrophy type 2 and Duchenne muscular dystrophy.

In a preferred embodiment for the first aspect and the second aspect of the invention, the boron compound is selected from the list consisting of Borax, 2-boronoethyl, 3-boronopropyl, m- and p boronophenyl and pinacolato thereof, 1-Propylboronic acid catechol ester, Ethyl dihydrogen borate, triethylene borate, 2-Aminoethyl diphenylborinate, 2-hydroxy-1,3,2-dioxaborolan-4-yl)methoxyl, 4-Hydroxy-3-methoxyphenylboronic acid pinacol ester, 2-((2- hydroxy benzo[d][1,3,2]dioxaborol-5-yl)oxyl, m-(boronooxy)phenoxyl, p-(boronooxy)phenoxyl and ((R)-3-Methyl-1-((S)-3-phenyl-2-(pyrazine-2-carboxamido)propanamido)butyl)boronic acid (also referred as Bortezomib), 5-Fluoro-1,3-dihydro-1-hydroxy-2,1-benzoxaborole (also referred as tavaborole), Benzonitrile, 4-[(1,3-dihydro-1-hydroxy-2,1-benzoxaborol-5-yl)oxy] (also referred as crisaborole).

These adjuvants allow the use of ultra-low doses of boron or boron compounds, improving the applicability of these combinations for a biomedical application, as they can enhance the effect of synergistic activation of the NaBC1 transporter and adhesion receptors in cells. Thus, the combination of the first aspect, improve myotube formation and myotube diameter as well as it suppressed cell mortality even in the higher concentrations.

The second aspect of the invention relates to a pharmaceutical composition comprising the combinations of the first aspect, thus comprising at least;
- A boron compound;
- at least an adjuvant which comprises fibronectin, cell adhesion peptide domains or mixtures thereof; provided that when the adjuvant is fibronectin, the boron compound is not borax; and
- optionally at least a pharmaceutical acceptable excipient.
- Optionally, when the adjuvant comprises fibronectin, the ratio of boron compound to the adjuvant is from 1.10⁻¹ to 1.10⁻⁶, preferably from 1.10⁻² to 1.10⁻⁶ by weight and/or wherein the adjuvant comprises cell adhesion peptide domains, the ratio of boron compound to the adjuvant is from 10 to 1.10⁻⁶ by weight, preferably from 5 to 1.10⁻⁶ by weight.

In a preferred embodiment of the composition of the second aspect, the cell adhesion peptide domain is a peptide which comprises or consists of a sequence selected from a list consisting of the SEQ ID NO: 1 (RGD), SEQ ID NO: 2 (IKVAV), SEQ ID NO: 3 (GRGDSPC) and SEQ ID NO: 4 (CSRARKQAASIKVAVSADR).

In a more preferred embodiment of the composition of the second aspect, the boron compound is bortezomib and the ratio of bortezomib to adjuvant is selected from 1.10⁻² to 1.10⁻⁶ by weight. Even more preferably, the adjuvant is a cell adhesion peptide domain.

The third aspect of the invention relates to a pharmaceutical composition comprising;
- A boron compound;
- at least an adjuvant which comprises fibronectin or cell adhesion peptide domains;
- a support which comprises a substrate selected from the list consisting of hydrogel matrices, glassware and silica nanoparticles; and
- Optionally at least a pharmaceutical acceptable excipient.

The presence of the support of the invention allows the reduction of the amount of boron compound even lower, than in the compositions of the second aspect. The presence of the above support enhances the effect of synergistic activation of the boron cell membrane transporter (NaBC1) and adhesion receptors in cells, in particular as suitable matrices for use in the medical and pharmaceutical field. More particularly, these compositions are suitable matrices for use in regenerative therapeutic treatments and represent a suitable system for the manufacturing of a pharmaceutical composition comprising the boron compounds of the invention. Preferably, regenerative therapeutic treatments are selected from muscle repair, muscle regeneration, as a consequence of an injury due to sport lesions, injuries in general or muscle defects naturally generated by aging.

The approach considered in the present invention allows the combined activation of NaBC1 integrins and GFRs (Growth Factor Receptors) without delivery of any agent but permanently stimulation of cell membrane receptors using the composition of the second aspect.

The inventors have demonstrated that boron transporter (NaBC1) activation triggers integrin binding, giving rise to a functional cluster that promotes intracellular signalling in cooperation with GFRs.

In a preferred embodiment of the third aspect, the composition comprises a support, wherein said support comprises a substrate selected from the list consisting of hydrogel matrices, glassware and silica nanoparticles. When the support is a hydrogel, this can be an alginate hydrogel or a hydrogel comprising PEG, wherein the PEG can have a Mw from 1 kD to 20 kD, measured by GPC or ¹H NMR.

Preferably, the support comprises a surface functionalized with a linker, wherein the linker is bonded to at least one boron compound. Even more preferably, the linker is selected from the list consisting of alginate, tetraethyleneglycol, pentaethyleneglycol, hexaethyleneglycol, heptaethyleneglycol, octaethyleneglycol, PEG having a molecular weight Mn from 200 to 2000, measured by GPC; preferably the linker is tetraethyleneglycol. In a preferred embodiment the ratio of Linkers to adjuvant may be from 20:1 to 2:1, more preferably from 10:1 to 3:1

The compositions of the third aspect have the advantage to allow the use of ultra-low doses of boron compounds, and present simultaneously boron and cell adhesion domains, allowing the applicability of these materials and its combination with boron compounds in the biomedical field. These compositions reduce significantly the toxicity of the boron compounds when used in mammals, while ensuring a good efficacy. This combination is also suitable to be used in the preparation of pharmaceutical compositions, preferably in the preparation of pharmaceutical compositions suitable for the treatment of pathophysiological condition which affects skeletal muscle, preferably said condition is a dystrophy, even more preferably wherein the dystrophy is selected from the list consisting of myotonic dystrophy type 1, myotonic dystrophy type 2 and Duchenne muscular dystrophy.

In a preferred embodiment of the third aspect, when the adjuvant comprises fibronectin, the ratio of boron compound to the adjuvant is from 1.10⁻¹ to 1.10⁻⁸, preferably from 1.10⁻² to 1.10⁻⁷ by weight and/or wherein the adjuvant comprises cell adhesion peptide domains, the ratio of boron compound to the adjuvant is 10 to 1.10⁻⁷, preferably from 5 to 1.10⁻⁶ by weight. Preferably, when the adjuvant comprises cell adhesion peptide domains and the ratio of boron compound to the adjuvant is from 5 to 1.10⁻⁷ by weight, more preferably from 1.10⁻¹ to 1.10⁻⁶ by weight. The aforementioned ranges allow the use of ultra-low doses of boron compounds, while improving the safety and ensuring efficacy. Ranges lower than the ones above will provide lower efficacy. Ranges higher than the ones above may increase toxicity and do not ensure efficacy.

In a preferred embodiment, the synthetic system materials of the third aspect comprise boron containing molecules covalently bound on a support, wherein the material is characterized by a surface functionalized with a linker which is bonded to at least one boron-containing compound. More preferably, the support comprises a substrate, said substrate can be selected from hydrogel matrices, in vitro culture plates, glassware and silica nanoparticles. The support which comprises a hydrogel matrix substrate enables a high level of control for delivering/presenting regenerative therapeutic treatments and represent a suitable system for the manufacturing of a pharmaceutical composition comprising the boron compounds of the invention. The in vitro culture surfaces can be used to advance research and understanding of cellular mechanisms related to boron transporter receptor and to develop in vitro models for drug screening.

In a preferred embodiment of the third aspect, the linker of the support is selected from the list consisting of tetraethyleneglycol, pentaethyleneglycol, hexaethyleneglycol, heptaethyleneglycol, octaethyleneglycol and polyethelene glycol (PEG) having a molecular weight Mn from 200 to 2000, measured by GPC. In an even more preferred embodiment, the linker is tetraethyleneglycol. The supports that contain a surface functionalized with the aforementioned linkers, provide stability, flexibility and hydration to favour interaction with cells.

In another preferred embodiment of the third aspect, the support is characterized by a surface functionalized with a linker, which allows the boron compound to either be permanently covalently bound to the substrate materials or be cleaved to be then released as a borate ion. In another preferred embodiment, the bond between the boron-containing compound and the linker is a hydrolysable bond or a covalent bond. In a more preferred embodiment, the bond between the boron-containing molecule and the linker is a covalent bond.

In a preferred embodiment for the third aspect of the invention, the boron compound is selected from the list consisting of 2-boronoethyl, 3-boronopropyl, m- and p boronophenyl and pinacolato thereof, 1-Propylboronic acid catechol ester, Ethyl dihydrogen borate, triethylene borate, 2-Aminoethyl diphenylborinate, 2-hydroxy-1,3,2-dioxaborolan-4-yl)methoxyl, 4-Hydroxy-3-methoxyphenylboronic acid pinacol ester, 2-((2- hydroxy benzo[d][1,3,2]dioxaborol-5-yl)oxyl, m-(boronooxy)phenoxyl, p-(boronooxy)phenoxyl, bis(pinacolato)diboron), ((R)-3-Methyl-1-((S)-3-phenyl-2-(pyrazine-2-carboxamido)propanamido)butyl)boronic acid (also referred as Bortezomib), 5-Fluoro-1,3-dihydro-1-hydroxy-2,1-benzoxaborole (also referred as tavaborole), Benzonitrile, 4-[(1,3-dihydro-1-hydroxy-2,1-benzoxaborol-5-yl)oxy] (also referred as crisaborole). More preferably, the boron compound is bortezomib and bis(pinacolato)diboron.

In another preferred embodiment of the third aspect of the present invention the surface of the support is further bonded to the adjuvant through the linker, preferably is further bonded through the same or through a different linker.

In another preferred embodiment of the third aspect of the present invention, the linker is also bonded to at least one boron-containing compound. More preferably, the bond between the boron compound and the linker is a hydrolysable bond or a covalent bond. In an even more preferred embodiment of the third aspect, the composition comprises a substrate functionalised with hydroxyl groups of TEG, previously reacted with *N*-Maleoyl-β-glycine and the appropriate cysteine terminated cell adhesion peptide domains, wherein the cell adhesion peptide domains comprise a sequence selected from a list consisting of SEQ ID NO: 1 (RGD), SEQ ID NO: 2 (IKVAV), SEQ ID NO: 3 (GRGDSPC) and SEQ ID NO: 4 (CSRARKQAASIKVAVSADR) or any combination thereof.

In another preferred embodiment, the composition of the third aspect, the composition comprises
- A boron compound from 5 to 1.10⁻⁶ % by weight, preferably from 2 to 1.10⁻⁵ %,
- at least an adjuvant which comprises fibronectin or cell adhesion peptide domains, in an amount from 5 to 1.10⁻⁶ % by weight; preferably from 2 to 1.10⁻³%
- a support which comprises a substrate selected from the list consisting of hydrogel matrices, glassware and silica nanoparticles, in an amount from 95-80 % by weight

In another preferred embodiment, the composition of the third aspect, the composition comprises
- A boron compound from 5 to 1.10⁻⁶ % by weight, preferably from 3 to 1.10⁻³ %,
- at least an adjuvant which comprises fibronectin or cell adhesion peptide domains, in an amount from 10 to 1.10⁻⁴ % by weight; preferably from 2 to 1.10⁻³ %
- a support which comprises a substrate selected from the list consisting of hydrogel matrices, glassware and silica nanoparticles, in an amount from 95-80 % by weight

In another preferred embodiment, the composition of the second and third aspects is an injectable pharmaceutical composition, preferably an injectable subcutaneous or intramuscular composition. Preferably, the injectable pharmaceutical composition is a lyophilized composition.

When the injectable pharmaceutical compositions of the second and third aspect can be formulated according to know methods in the art, using suitable diluents or/and suitable non-toxic solvents for parenteral administration, such as mannitol, 1 ,3-butanodiol, water, Ringer solution or isotonic sodium chloride solution, dispersion or suspension dispersion agents, or humidifying agents, such as sterile oils, including mono- or synthetic diglycerides, fatty acids or oleic acid.

In another particular embodiment, the composition of the second and third aspects is an oral composition in the form of a tablet, capsule, cream, gel, liquid or a powder or a topical composition in the form of a cream, ointment, gel, liquid or a dispersible powder.

The compositions of the present invention may also include at least one excipient. The at least one excipient may be chosen, for example, from surfactants (cationic, anionic, or neutral), surface stabilizers, and other enhancers, such as preservatives. Non-limiting examples of surfactants that may be used in accordance with the present disclosure include non-ionic surfactants such as a polysorbate surfactant (e.g., polysorbate 20 (Tween 20^{™}), and polysorbate 80 (Tween 80^{™})). In some embodiments, the compositions of the present disclosure contain multiple pH stabilizers to form a pH buffering system within the composition. As an example of a preservative that may be added to the compositions of the present disclosure, non-limiting mention is made of glycerol, which may act as a preservative at certain concentrations. The compositions of the present disclosure may also include at least one emulsifier. Non-limiting examples of suitable emulsifiers include phospholipids, propylene glycol, polysorbate, poloxamer, and glyceryl monostearate. Other known pharmaceutical emulsifiers may be used.

The compositions of the present invention may be in any form suitable for topical application to the skin. For example, the compositions may be in the form of a solution such as a hydrogel, a tincture, a cream, an ointment, a gel, a lotion, and/or an aerosol spray.

The fourth aspect of the present invention relates to the combination of the first aspect and the composition of the second aspect for use in a method to improve muscle regeneration.

The fifth aspect of the present invention relates to the combination of the first aspect and the composition of the second aspect for use in a method of treatment or therapy of pathophysiological condition which affects skeletal muscle, preferably said condition is a dystrophy, preferably, the dystrophy is selected from the list consisting of myotonic dystrophy type 1, myotonic dystrophy type 2 and Duchenne muscular dystrophy.

The sixth aspect of the present invention relates to the composition of the second and third aspect for use in a method of treatment or therapy of pathophysiological condition which affects skeletal muscle, wherein said method comprises administering the boron to said mammal in an amount effective for decreasing muscle atrophy and/or in amount effective to increase muscle growth in said mammal. Preferably, the boron compound is bortezomib and is administered to a human in an effective dose from 1.10⁻⁶ to 1 mg once or twice weekly, preferably, from 1.10⁻⁴ to 0.5 mg once or twice weekly or from 1.10⁻² to 0.1 mg once or twice weekly.

The composition of the second and third aspect can be administered parenterally to humans, wherein the amount of the boron compound in the composition ranges from 1.10⁻⁷ to 1 mg, preferably from 1.10⁻⁶ to 0.5 mg or from 1.10⁻⁶ to 1.10⁻³ mg.

The seventh aspect of the present invention relates to the combination of the first aspect and the compositions of the second and third aspect for use as regenerative therapeutic treatments selected form the list consisting of, muscle repair, muscle regeneration, as a consequence of an injury due to sport lesions, injuries in general or muscle defects naturally generated by aging.

The eighth aspect of the present invention relates to the use of the compositions of the second and third aspect as cell culture substrates for muscle cell biopsies from patients, preferably, for muscle cell dystrophic biopsies or an immortalised dystrophic cell lines.

The ninth aspect of the present invention related to relates to a method to prepare the composition of the second aspect, comprising at least the steps of:
- Providing the boron compound;
- Mixing the boron compound with the adjuvant in a ratio of boron compound to the fibronectin from 1.10⁻¹ to 1.10⁻⁶, preferably from 1.10⁻² to 1.10⁻⁶ by weight and/or wherein the adjuvant comprises cell adhesion peptide domains, the ratio of boron compound to the adjuvant is from 10 to 1.10⁻⁶, preferably from 5 to 1.10⁻⁶ by weight; and
- Optionally adding a pharmaceutical acceptable excipient.

The tenth aspect of the present invention relates to a method to prepare the composition of the third aspect comprising a functionalised support, the method comprises at least the steps of:
a) Preparing the functionalised support by chemically modifying the surface of the support by silanization and further reacting with a compound having a terminal alkyne group;
b) Incorporating the boron compound in the functionalised support of the previous step by performing a coupling reaction on the product obtained in the previous step using a Pd-mediated cross-coupling agent to incorporate the boron compound into the linker, wherein the linker chain comprises a terminal azide group;
c) azide-alkyne click coupling between the modified surface from step (a) and the boron-containing linker from step (b);
d) performing a maleimide-thiol coupling between the support obtained in step (c) and adjuvant, wherein the adjuvant contains the appropriate cysteine-terminated peptides containing specific integrin adhesion sequences, preferably wherein the adjuvant is Fibronectin or a Cell adhesion domain peptide, which comprises or consists of a sequence selected from a list consisting of the SEQ ID NO: 1, SEQ ID NO: 2, SEQ ID NO: 3 and SEQ ID NO: 4;

### Examples

### Control samples without boron compounds

As controls, samples comprising Fibronectin coated glass substrates (FN-Coated Glass) were tested using two different muscular dystrophy human cell lines (DMD and DM1) and wild type cell (WT) in vitro.

DMD and DM1 were tested as dystrophic cells presenting hallmark features of the disease meaning reduced fusion capability and smaller and thinner myotubes compared to healthy controls. For the controls no boron compound was added.

### Preparation of the cell culture for both control and boron samples

Briefly, the aforementioned cells (DMD, DM1 and WT) were cultured and maintained in 75 cm² flasks with skeletal growth medium (Promocell skeletal muscle cell basal medium supplemented with growth factors, calf serum and P/S 1%) at 37 °C in a humidified atmosphere of 5 % CO2, until 80% confluence. Glass cover slip substrates (12 mm diameter) were sterilised with UV light for 30 min and then coated with 3.6 µg of fibronectin (FN). After incubation with the FN coating for 1 h at RT, cells were then seeded at 40.000 cells/well (p-24 plate) in skeletal muscle cell basal medium.

For the examples where a boron compound was added, after the previous step, different amounts of the boron compound were added in the culture medium, Promocell skeletal muscle cell basal medium).

After 24 h of culture growth medium was changed by myoblast differentiation medium (DMEM high glucose/P/S 1%, insulin 10 µg/mL) and for the examples where the boron compound was added, then different amounts of boron compound were added. Differentiation medium was changed every 2 days and if necessary, the same amount of the boron compound, as previously incorporated was also added in every change. Myotube formation was evaluated after 8 days. Cells were fixed with formalin 4% at the end of the culture for 30 minutes at 4° C. After fixing, cells were washed and conserved at 4° C in DPBS. Starting with the fixed cells preserved at 4° C in DPBS, samples were then rinsed with DPBS and permeabilised with DPBS/0.5% Triton X-100 at room temperature for 5 min. Samples were then incubated with sarcomeric α-actinin primary antibody (abcam, 1:200), muscle marker for immunofluorescence detection, in blocking buffer DPBS/2% BSA at 37 °C for 1 h or overnight at 4 °C. The samples were then rinsed twice in DPBS/0.1% Triton X-100 and incubated with the secondary antibody anti mouse Cy3 (Invitrogen, 1:200) in blocking buffer at room temperature for 1 h. Finally, samples were washed twice in DPBS/0.1% Triton X-100 before mounting with Vectashield containing DAPI (Vector Laboratories) and observed under an epifluorescence microscope (Nikon Eclipse 80i).

### Example 1: Combination of the adjuvant fibronectin and the boron compound, bortezomib,

### Example 1.1, Example 1.2, Example 1.3 and Example 1.4

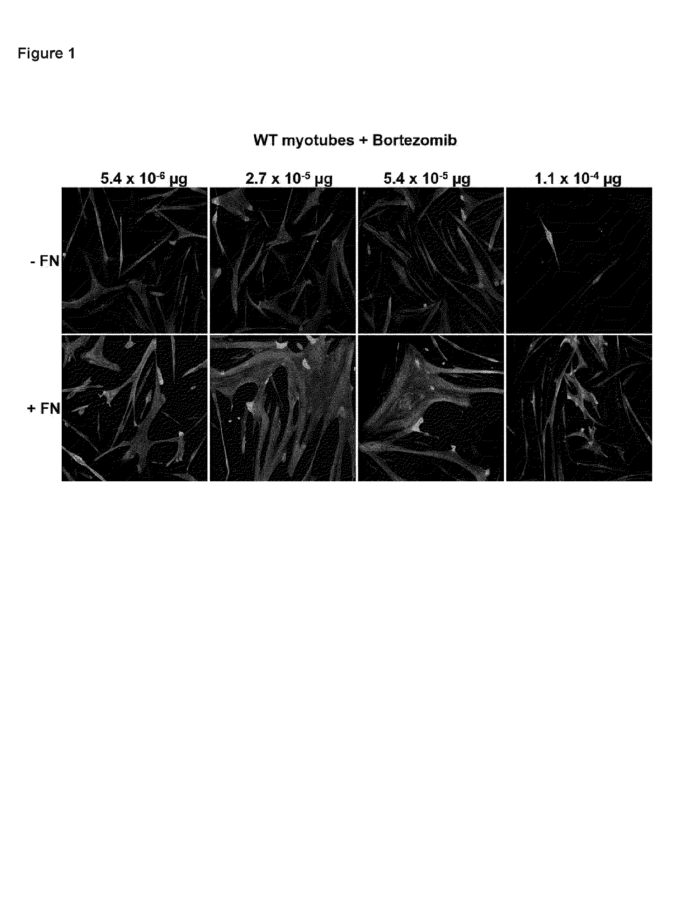

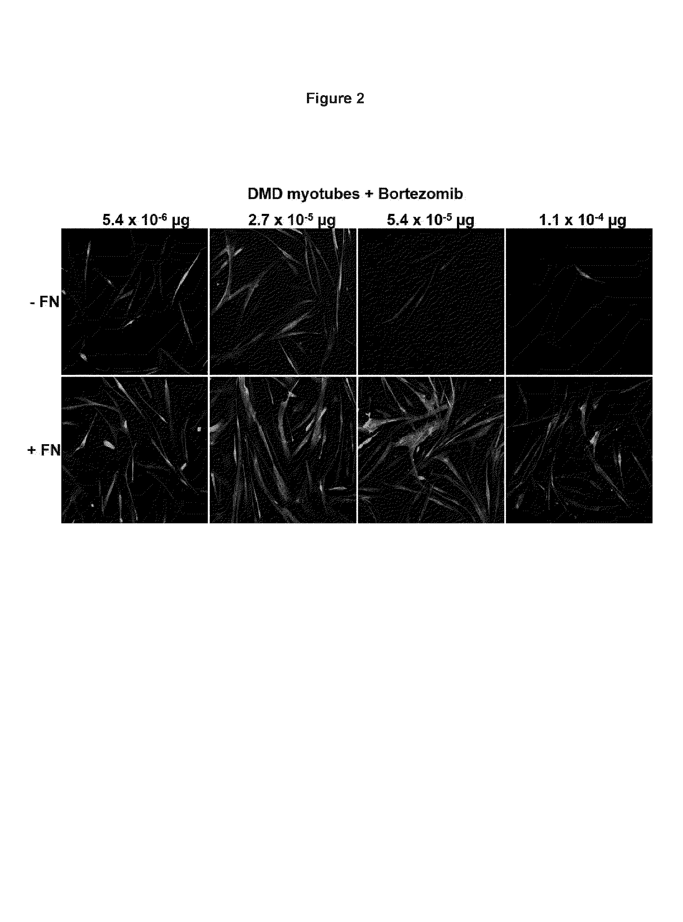

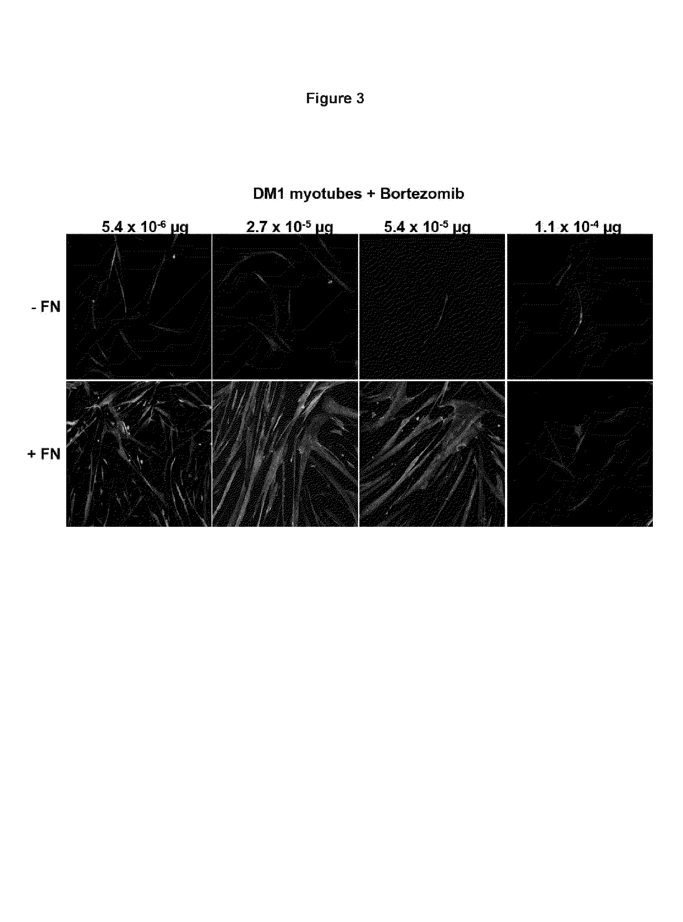

A glass substrate was coated with Fibronectin (FN-coated glass). A medium using 2 different muscular dystrophy human cell lines (DMD and DM1) and also in wild type cells (WT), in vitro, were prepared using different amounts of bortezomib, 5.4 10⁻⁶ µg (0.0000054 µg) Ex 1.1, 2.7 10⁻⁵µg (0.000027 µg) Ex 1.2, 5.4 10⁻⁵ µg (0.000054 µg) Ex 1.3 and 1.1 10⁻⁴µg (0.00011 µg) Ex 1.4, (all non-cytotoxic concentrations) in the culture medium together with fibronectin (3.6 µg) and was deposited on the FN-coated glass. Results of the combination of bortezomib with adjuvant at different concentrations using different cell mediums, WT, DMD and DM1, can be seen in fig 1, 2 and 3, respectively.

Cells were cultured and maintained in 75 cm² flasks with skeletal growth medium (Promocell skeletal muscle cell basal medium supplemented with growth factors, calf serum and P/S 1%) at 37 °C in a humidified atmosphere of 5 % CO₂, until 80% confluence. Glass cover slip substrates (12 mm diameter) were sterilised with UV light for 30 min and then coated with 3.6 µg of fibronectin (FN). After incubation with the FN coating for 1 h at RT, cells were then seeded at 40,000 cells/well (p-24 plate) in Promocell skeletal muscle cell basal medium and addition of the different amounts of bortezomib 0.0000054 µg, 0.000027 µg, 0.000054 µg and 0.00011 µg in the culture medium.

After 24 h of culture, growth medium was changed by myoblast differentiation medium (DMEM high glucose/P/S 1%, insulin 10 µg/mL) and the different amounts of bortezomib, 5.4 10⁻⁶ µg, 2.7 10⁻⁵ µg, 5.4 10⁻⁵ µg and 1.1 10⁻⁴ µg, were added to each sample. Differentiation medium was changed every 2 days and the different amounts of bortezomib 5.4 10⁻⁶ µg, 2.7 10⁻⁵ µg, 5.4 10⁻⁵ µg and 1.1 10⁻⁴ µg, were added to each sample in every change. Myotube formation was evaluated after 8 days. Cells were fixed as described in the control samples. Afterwards, the samples were also rinsed and washed as described in the control samples.

Surprisingly, the addition of bortezomib in the culture medium not only enhanced myotube differentiation but also importantly increased myotube diameter. Considering that, dystrophic cells present hallmark features of the disease meaning reduced fusion capability and smaller and thinner myotubes compared to healthy controls, it was concluded that the effect of bortezomib together with FN restored myotube formation (fusion capability) and improved myotube diameter, indicative of myotube physiological function repair. This led to a significant reduction in the use of bortezomib dosages, compared to the usual bortezomib dosages and even versus other borax concentrations used in previous works, providing significant effects in myotube restoration using ultra-low doses of this boron compound (bortezomib), as seen in table 1.

**Table 1: Percentage of differentiation and myotube diameters of WT, DM1 and DMD cells cultured for 8 days without or with fibronectin and different concentrations of bortezomib.**

| % of cell differentiation and myotube diameter (µm) after using different bortezomib concentrations. | | | | | | | | |
|---|---|---|---|---|---|---|---|---|
| Type of cells | 5.4 10⁻⁶ µg (0.0000054 µg) | | 2.7 10⁻⁵ µg (0.000027 µg) | | 5.4 10⁻⁵ µg (0.000054 µg) | | 1.1 10⁻⁴ µg (0.00011 µg) | |
| WT | 47% | 32 µm | 55% | 60 µm | 45% | 48 µm | 0% | 0 µm |
| WT-FN | 70% | 122 µm | 79% | 319 µm | 72% | 270 µm | 39% | 63 µm |
| DM1 | 14% | 11 µm | 13% | 11 µm | 0.5% | 4 µm | 1% | 4 µm |
| DM1-FN | 37% | 44 µm | 55% | 79 µm | 54% | 130 µm | 1% | 23 µm |
| DMD | 9% | 15 µm | 31% | 27 µm | 5% | 5 µm | 1% | 2 µm |
| DMD-FN | 47% | 45 µm | 80% | 72 µm | 61% | 46 µm | 17% | 17 µm |

Considering that, dystrophic cells present hallmark features of the disease meaning reduced fusion capability and smaller and thinner myotubes compared to healthy controls. It was concluded that the effect of bortezomib together with FN help restore myotube formation (fusion capability) and myotube diameter, indicative of myotube physiological function repair.

The same cell lines (DMD and DM1) as well as WT tested with bortezomib together with fibronectin strongly induced myotube formation in WT, DMD and DM1 after 8 days of culture (Figure 1), when compared to those without fibronectin.

### Example 2: Combination of borax and the adjuvant "cell adhesion domain peptide SEQ ID NO: 1 (RGD)".

A glass substrate was coated with 12.43 µg cell adhesion domain peptide SEQ ID NO: 1 (RGD) using 2 different muscular dystrophy human cell lines (DMD and DM1) and also in wild type cells (WT) in vitro. Afterwards, borax was added to the culture medium, previously prepared, using different amounts of the boron compound, 12.76 µg (Ex 2.1) and 31.78 µg (Ex 2.2) (non-cytotoxic concentrations) and was deposited on the RGD-coated glass following the same procedure as described in example 1.

### General synthetic strategy for the preparation of supports with TEG linkers

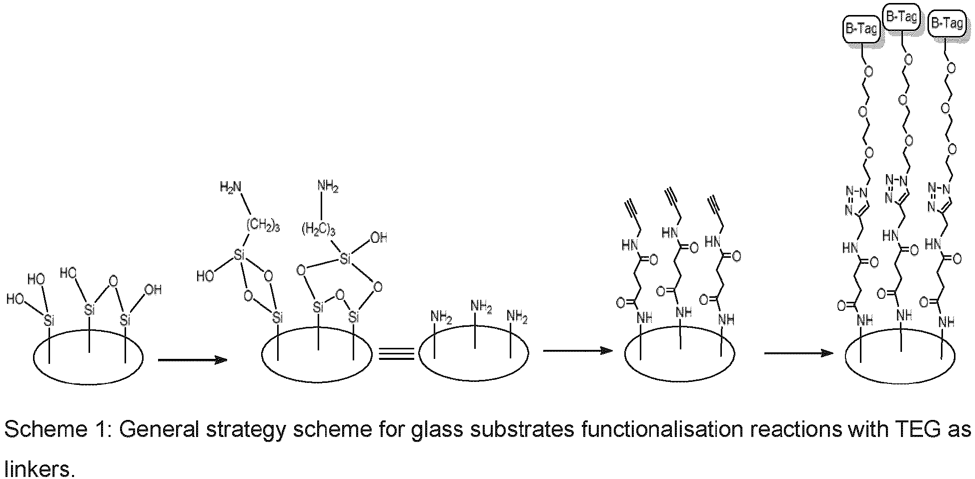

The compositions comprising a support, and adjuvant and a boron compound, which can be permanently covalently bound to the support materials or cleaved on them, allow the release of the boron compound.

The presence of the linker provides stability, flexibility and hydration to favour interaction with cells.

### Example 3: General procedure for the preparation of modified of glass supports

The procedure is based on the following methodology for 12 mm diameter glass cover slides, which was adapted to bigger glass covers when needed. Briefly, except when otherwise noted, all reactions were carried out at 37° C under ecotron shaking and are optimized for 50 glass units:
1) Acid washing of glass covers: Employing a 500 mL Erlenmeyer, glass covers are acid washed with piranha solution (200 mL 3:1 H₂O₂ 30% / H₂SO₄) with gently shaking during 30 min. Excess reagents were washed out with 3 x 500 mL bidistilled water. Glass cover slides were then dried under vacuum at 80° C for overnight (o/n).
2) Silanization with APTES was carried out solution of APTES (5 mL) in EtOH (50 mL) for 48 h. Excess reagents were washed out with 3 x 200 mL EtOH.
3) Remainder EtOH was removed with 3 x 100 mL Dimethylformamide (DMF) washes. Then there was accomplished the reaction with 9 (150 mg) employing 1-Ethyl-3-(3-dimethylaminopropyl)carbodiimide (EDC) (240 mg) and N,NDiisopropylethylamine (DIPEA) (440 µL) in DMF (100 mL) for 24 h. Excess of reagents were washed out with 2 x 100 mL DMF and 3 x 100 mL EtOH.
4) The Cu-catalyzed click coupling was then done by reacting the glass covers with a solution of CuSO₄·5H₂O (10 mg), sodium ascorbate (1.2 g) and either 5, 8 or 12 (200 mg) in EtOH:H₂O (1:1, 100 mL) for 24 h. Excess of reagents were removed with 2 x 100 mL H₂O and 3 x 100 mL EtOH washings.

### Example 4: Composition of the adjuvant fibronectin with the boron compound bis(pinacolato)diboron, covalently anchored to a support comprising TEG linkers

A glass substrate acting as a support was chemically modified for the anchoring of TEG linkers with a boron compound covalently bound (bis(pinacolato)diboron). Afterwards, the substrate was coated with 3.6 µg of fibronectin using 2 different muscular dystrophy human cell lines (DMD and DM1) and also in wild type cells (WT) in vitro. Both TEG and TEG-B platforms strongly enhance myotube formation and diameter, accelerating muscle regeneration as shown in the reduction of the time of culture of the cells, 6 days compared to 8 days (Example 1).

**Table 2: Percentage of differentiation and myotube diameters of human WT, DM1 and DMD cells cultured for 6 days onto FN-coated supports presenting tetraethylenglycol chains (TEG) chemically anchored on a glass surface, or TEG-B covalently bound to the boron compound.**

| % of cell differentiation and myotube diameter (µm) | | | | |
|---|---|---|---|---|
| Type of cells | TEG | | TEG-B | |
| WT | 55 % | 145 µm | 68 % | 207 µm |
| DMD | 62 % | 112 µm | 79 % | 244 µm |
| DM1 | 42 % | 144 µm | 52 % | 188 µm |

### Example 5: General synthetic strategy for the preparation of supports with TEG moieties and TEG linkers

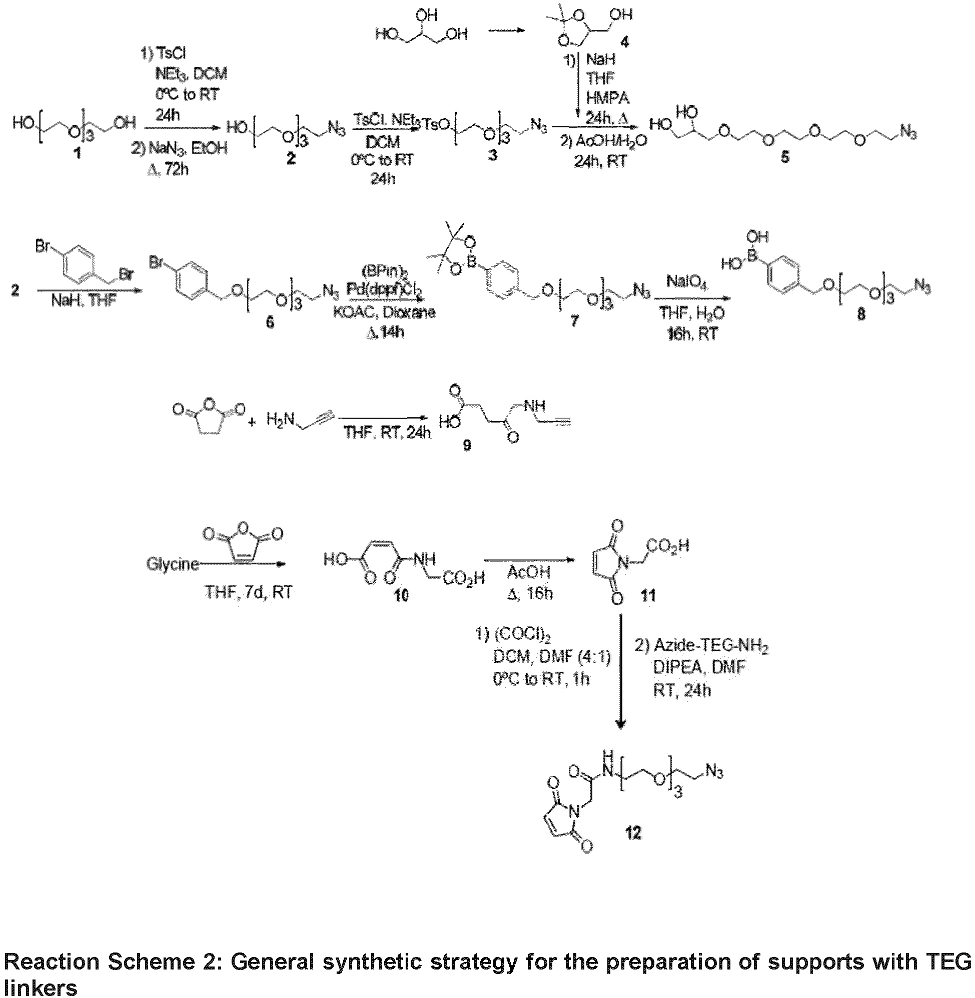

***Preparation of 1-azido-3,6,9-trioxadodecan-12-ol (N3-TEG-OH, 2)*.** Compound **2** was obtained throughout a two-step procedure. First, tetraethylene glycol (100 mL, 0.58 mol) and triethylamine (60mL, 0.43 mol) were dissolved in 250 mL of anhydrous Dichloromethane (DCM) under nitrogen. Then, after cooling to 0° C pTsCI (58 g, 0.30 15 mol) was added to the mixture, which was reacted for 24 h at room temperature (RT). When the reaction finished, the reaction was filtered out from the solids and the organic layer poured into water (150 mL). The organic layer was separated, and the aqueous layer extracted with DCM (3 x 100mL), the resulting combined organic layers were washed with water and brine and dried over MgSO₄. After evaporation, monotosylate was obtained as yellow oil (88 g, 84%) and used for the next step without further purification. ¹HNMR (CDCl₃, 250 MHz), δ(ppm): 7.78 (d, J = 8.4 Hz, 2H), 7.33 (d, J = 8.5 Hz, 2H), 5 4.16 (t, J = 5.7Hz, 2H), 3.75-3.57 (m, 14H), 2.44 (s, 3H).

Then, to afford title compound, the monotosylate (88 g, 0.25 mol) was reacted together with NaN₃ (40.6 g, 0.63 mol) in EtOH (100 mL) at reflux and under inert atmosphere for 72 h. When the reaction was completed, the precipitate was filtered off and the solvent evaporated. The residue dispersed in silica gel and the product purified employing a flash column chromatography using EtOAc as mobile phase.

Product 2 was obtained as yellow oil (44 g, 85%). ¹HNMR (CDCl₃, 250 MHz), δ(ppm): 3.60-3.74 (m, 14H), 3.57 (t, J = 5.6 Hz, 2H), 3.35 (t, J = 5.6 Hz, 2H). MS(ESI+), m/z calculated for (M+H)+ [C8H18N3O4]+: 220.13, found: 220.2.

**Preparation of 12-azido-3,6,9, trioxadodecanyl-p-toluenesulfonate (N3-TEG-OTs, 3).** To an ice cooled solution of NEt3 (9.5 mL, 67 mmol) and 2 (10 g, 45 mmol) in DCM (100 mL), was added p-toluenesulfonyl chloride (9.1 g, 47 mmol). The reaction was stirred at 0° C for 1 h and overnight at RT. Once completed, the reaction was diluted with DCM (100 mL), washed with NaHCO₃, water and brine. Then the organic layer was dried over Na₂SO₄, filtered, and purified by SiO₂ flash chromatography using EtOAc Heptane (1:1) to EtOAc as mobile phase. Title compound was obtained as a clear oil. (6.9 g, 76% yield). ¹HNMR (CDCₗ₃, 250 MHz), δ (ppm): 7.78 (d, J = 8.4 Hz, 2H), 7.33 (d, J = 8.5 Hz, 2H), 4.22 - 4.08 (m, 2H), 3.71 - 3.62 (m, 4H), 3.59 - 3.56 (m, 4H), 2.44 (s, 3H). MS(ESI+), m/z calculated for (M+H)+ [C₁₅H₂₄N₃O₆S]+: 374.14, found: 374.1.

**Preparation of (2,2-dimethyl-[1,3]-dioxolan-4-yl)metanol (Solketal, 4).** This compound was synthesized according to the following procedure. Briefly, 7.5 g (81.44 mmol) of glycerol were dissolved in 300 mL of acetone into a 500 mL round bottom flask. To it there was added a catalytic amount of iodine (0.75 g, 2.95 mmol). The reaction was allowed to evolve for 5 days at RT. Once completed, the resulting mixture was concentrated under reduced pressure, diluted with ethyl acetate and washed with 2 x 30 100 mL sodium thiosulfate (10%) and brine. Finally, upon drying over MgSO₄ and evaporation under vacuum, the product was obtained as a colorless oil (9 g, 68.1 mmol, 84%). ¹HNMR (250 MHz, CDCl₃) δ 7.18 - 7.00 (m, 1H), 4.21 (tdd, J = 6.5, 5.3, 3.9 Hz, 1H), 4.01 (dd, J = 8.2, 6.6 Hz, 1H), 3.75 (dd, J = 8.2, 6.5 Hz, 1H), 3.69 (dd, J = 12.1, 4.3 Hz, 1H), 3.56 (dd, J = 11.6, 5.2 Hz, 1H), 1.41 (s, 3H), 1.34 (s, 3H). MS(ESI+), m/z calculated for (M+H)+ [C6H13O6]+: 133.09, found: 133.0.

**Preparation of 1-azido-3,6,9,12-tetraoxapentadecane-14,15-diol (N3-TEG-Diol, 5).** The title compound was prepared following a two-step procedure. First, a mixture of racemic solketal 4 (1.3 g, 9.5 mmol) and Hexamethylphosphoramide 5 (HMPA) (17 ml) in anhydrous Tetrahydrofuran (THF) (50 mL) was added to a suspension of NaH 60% (0.94 g; 23.6 mmol) in anhydrous THF (18 mL) under argon. The resulting mixture was reacted at RT for 1 h. Then, onto the former solution, there was slowly added a solution of 3 (3.5 g; 11.8 mmol) in anhydrous THF (15 mL); the resulting reaction mixture was heated to reflux for 24 h. Once when finished, the mixture was cooled down and was quenched with water (30 mL) and washed with EtOAc three times. The combined organic layers were washed twice with a 1 M HCI solution, twice with a saturated NaHCO₃ solution and brine; then were dried over MgSO₄ and evaporated to dryness. The crude was purified by flash chromatography on silica gel with a Heptane/EtOAc (1:1) mixture as mobile phase. The intermediate acetal compound was obtained as a colorless oil (1.4 g; yield: 47%). ¹HNMR (250 MHz, CDCl₃) δ 4.34 - 4.21 (m, 1H), 4.05 (dd, J = 8.3, 6.4 Hz, 1H), 3.72 (dd, J = 7.6, 6.6 Hz, 1H), 3.69 - 3.61 (m, 14H), 3.57 (dd, J = 10.0, 5.7 Hz, 1H), 3.49 (dd, J = 10.0, 5.5 Hz, 1H), 3.38 (t,J = 5.1 Hz, 2H), 1.41 (s, 3H), 1.35 (s, 3H). MS(ESI+), m/z calculated for (M+Na)+ [C₁₄H₂₇N₃O₆Na]+: 356.2, found: 356.2. On the second step the acetal was deprotected by treating the previous compound into an AcOH-H₂O mixture (1:1, 10 mL) during 24 h at RT. The final product was obtained upon evaporation of volatiles with the aid of toluene for the remainder moisture. Title compound was obtained as a clear oil (1.2 g, yield: 96%). ¹HNMR (250 MHz, MeOD) δ 4.64 (bs, 6H), 3.84 - 3.71 (m, 1H), 3.70 - 3.60 (m, 11H), 3.60 - 3.42 (m, 3H), 3.37 (dd, J = 8.8, 4.1 Hz, 2H). MS(ESI+), m/z calculated for (M+H)+ [C₁₁H₂₄N₃O₆]+: 294.2, found: 294.3.

**Preparation of 13-azido-1-(4-bromophenyl)-2,5,8,11-tetraoxatridecane (N3-TEG-Br, 6).** To an icecold suspension of hexane-washed sodium hydride (60% in mineral oil, 0.42 g, 10.5 mmol) in anhydrous THF (5 mL) was added a solution of N₃-TEG-OH (2, 2.2 g, 30 mmol) under nitrogen atmosphere. The mixture was stirred at 0° C for 1 h, followed by a dropwise addition of a solution of 4-bromobenzyl bromide (3.0 g, 12 mmol) in dry THF (5 mL). The resulting mixture was stirred at 0° C for an additional 1 h, allowed to warm up to RT and stirred overnight. Once completed, the mixture was cooled to 0° C and quenched with saturated ammonium chloride (50 mL), and extracted with EtOAc (3 x 200 mL). The combined organic layer was dried over MgSO₄, concentrated and purified by flash column chromatography using silica gel as adsorbent and EtOAc/Heptane (2:1) as eluent. The title compound was obtained as a colorless oil. (2.0 g, 51 %). ¹HNMR (250 MHz, CDCl₃) δ 7.45 (d, J = 8.4 Hz, 2H), 7.21 (d, J = 8.5 Hz, 2H), 4.50 (s, 2H), 3.70 - 3.58 (m, 14H), 3.41 - 3.33 (m, 2H). MS(ESI+), m/z calculated for (M+Na)+ [C15H2281BrN3O4Na]+: 410.07, found: 410.2.

**Preparation of 2-(4-(13-azido-2,5,8,11-tetraoxatridecyl)phenyl)-4,4,5,5-tetramethyl-1,3,2-dioxaborolane (N3-TEG-BPin, 7).** Into a flame-dried rounded bottom flask fitted with a reflux condenser is prepared a solution of compound 6 (1.92 g, 4.95 mmol), bis(pinacolato)diboron (1.89 g, 7.4 mmol), potassium acetate (1.45 g, 14.8 mmol), Pd(dppf)Cl2 (181 mg, 5 mol%) and dry 1,4-dioxane (50 mL). This mixture is degassed and flushed with nitrogen for 3 times and then heated at 110° C for 24 h. After cooling to RT, DCM (15 mL) was added and the resulting mixture was filtered through a celite pad, evaporated and purified by flash column chromatography using silica gel as adsorbent and EtOAc/Heptane (1:1) as eluent. Title compound was obtained as a colorless oil. (1.66 g, 77 %). ¹HNMR (250 MHz, CDCl₃) δ 7.78 (d, J = 7.9 Hz, 2H), 7.34 (d, J = 7.8 Hz, 2H), 4.58 (s, 2H), 3.75 - 3.58 (m, 14H), 1.26 (s, 6H), 1.25 (s, 6H). MS(ESI+), m/z calculated for (M+Na)+ [C21H34BN3O6Na]+: 458.24, found: 458.2.

**4-(13-azido-2,5,8,11-tetraoxatridecyl)phenylboronic acid (N3-TEG-B(OH)2, 8).**
Into a round bottom flask is prepared a solution of compound 7 (0.5 g, 1.15 mmol) into an acidified mixture of THF and H₂O (4:1, 50 mL plus 0.1 mL concentrated HCI) to which is added solid NalO₄ (734 mg, 3.45 mmol). The reaction is maintained for overnight at RT. Once completed the solvents are removed under vacuum and the product purified by flash column chromatography using silica gel as adsorbent and EtOAc as eluent. Title compound was obtained as a colorless oil (398 mg, 98%). ¹HNMR (250 MHz, CDCl₃) δ 7.77 (d, J = 7.8 Hz, 2H), 7.36 (d, J = 7.8 Hz, 2H), 4.59 (s, 2H), 3.77 - 3.52 (m, 14H). MS(ESI+), m/z calculated for (M+H)+ [C15H25BN3O6]+: 354.18.24, found: 354.2.

**4-oxo-5-(prop-2-yn-1-ylamino)pentanoic acid (9)** was prepared directly from acylation of propargylamine (281 µL, 4.4 mmol) with succinic anhydride (400 mg, 4 mmol) in dry THF (15 mL). To achieve the reaction both reagents were dissolved in THF and were reacted for 24 h at RT. When finished, the solvent and excess of amine were evaporated under vacuum and the residue triturated with chloroform to provide a brown solid (649 mg, 96%). ¹HNMR (250 MHz, DMSO) δ 8.30 (t, J = 5.1 Hz, 1H), 3.83 (dd, J = 5.4, 2.5 Hz, 2H), 3.09 (t, J = 2.5 Hz, 1H), 2.41 (dd, J = 10.7, 4.2 Hz, 2H), 2.30 (dd, J = 10.6, 4.1 Hz, 2H). MS(ESI-), m/z calculated for (M-H)- [C8H10NO3]-:168.07, found: 168.1.

**(Z)-4-((Carboxymethyl)amino)-4-oxobut-2-enoic acid (10).** The reaction between the maleic anhydride (5 g, 5 51 mmol, 1.0 equiv) and glycine (3.83 g, 51 mmol, 1.0 equiv) was carried out in THF allowing the mixture to react for 7 days. The resulting precipitate was collected by filtration, and washed with THF to provide the 8.72 g of the acid (99%) as a white solid. ₁HNMR (250 MHz, DMSO-d6) δ 9.31 (bs, 1H), 6.40 (d, J = 12.4 Hz, 1H), 6.29 (d, J = 12.4 Hz, 1H), 3.89 (d, J = 4.9 Hz, 2H).

2-(2,5-dioxo-2,5-dihydro-1H-pyrrol-1-yl)acetic acid (11) was obtained upon cyclization of 10 in acetic acid (80 mL) under reflux for 16 h. The reaction mixture was evaporated and purified by SiO2 flash column chromatography employing acetone- DCM (1:4) as mobile phase. Desired compound was obtained as a white solid (1.80 g, 11.7 mmol, 23%). ¹HNMR (250 MHz, DMSO-d6) δ 7.13 (s, 2H), 4.14 (s, 2H). HRMS (EI), m/z calculated for C6H5NO4 155.0226 (M+), found 155.0230.

**N-(2-(2-(2-(2-azidoethoxy)ethoxy)ethoxy)ethyl)-2-(2,5-dioxo-2,5-dihydro-1Hpyrrol-1-yl)acetamide (N3-TEG-Mal, 12).** Compound 12 was prepared by direct acylation of the commercial bifunctional amino-azide tetraethyleneglycol (N3-TEGNH2).
First compound 11 was transformed into is acyl chloride. Briefly, 155 mg (1 mmol) were dissolved in a 4 to 1 mixture of dry DCM and DMF (15mL) at 0°C and under inert atmosphere. Then, to the mixture was dropwise added oxalyl chloride (2M in DCM, 0.5mL). The reacting mixture was then removed from the cooling bath and stirred at RT for 1 h. Once completed, to this mixture was added a solution of aminoazide tetraethylene glycol (229 mg, 1.05 mmol) and DIPEA (366 µL, 2.1 mmol) in dry DMF (5 mL). The mixture was then reacted for 24 h at RT. Final product was purified after evaporation of solvents and purification by SiO₂ flash column chromatography employing EtOAc/Heptane (1:2) as mobile phase and was obtained as a clear oil. (256 mg, 72% yield). ¹HNMR (CDCl3, 250 MHz), δ (ppm): 7.62 (s, 2H), 4.20 - 4.05(m, 4H), 3.77-3.59 (m, 12H), 3.35 (t, J = 5.6 Hz, 2H). MS(ESI+), m/z calculated for (M+H)+ [C14H22N5O6]+: 356.16, found: 356.1.

### Example 6: General Procedure for the preparation of hydrogels supports having a functionalized surface with TEG linkers to provide composition comprising a boron compound, an adjuvant (fibronectin or cell adhesion domains) and a hydrogel support comprising TEG as linkers.

The procedure described is suitable for both different types of PEG hydrogels and dialysis must be adapted to the final molecular weight of the chosen hydrogel. Herein both alginate and PEG hydrogels could be modified with carboxylate or amino groups in their respective structures.
1) Direct amidation of carboxylate-modified hydrogels with amino-containing peptides (for instance cell adhesion domains or Fibronectin) was accomplished by reacting a solution of hydrogel in pH=7.4 10mM phosphate buffer (5mL) with the corresponding peptide (1 equiv), EDC·HCl (1.2 equiv) and sulfo-NHS (1.3 equiv) for 24 h at room temperature.
2) If needed, a transformation of carboxylate-containing hydrogels into amino modified hydrogels could be carried out. For so, it was reacted a solution of hydrogel in pH=7.4 10mM phosphate buffer (5mL) with the 1,6- hexamethylenediamine (1 equiv), EDC·HCl (1.2 equiv) and sulfo-NHS (1.3 equiv) for 24 h at RT. Removal of remainder reagents was achieved upon two 24 h dialysis cycles employing a 7KDa cut-off membrane (See step 5).
3) The functionalization of amino-modified hydrogels with thiol-containing peptides (for instance, cell adhesion domains or Fibronectin) was carried out employing a water-soluble specific bifunctional linker (Maleimide- PEG6-NHS ester). Briefly, onto a solution of hydrogel in pH=7.4 10mM phosphate buffer (5mL) were added the Maleimide-PEG6-NHS ester (1 equiv) and the corresponding peptide (1.2 equiv), allowing to react for 24 h at RT.
4) For the functionalization of amino-modified hydrogels with B-containing compounds, a water-soluble specific Cu-free bifunctional linker (DBCO-PEG4- NHS ester) was employed. Briefly, onto a solution of hydrogel in pH=7.4 10mM phosphate buffer (5mL) were added the DBCO-PEG4-NHS ester (1 equiv) and either 5 or 8 (1.5 equiv), allowing to react for 24 h at RT.
5) Purification of hydrogels was accomplished by dialysis employing a 7KDa cut-off membrane into 250 mL bidistilled water under gently shaking. 24 h washing steps were repeated twice in order to remove small, unreacted reagents. Once washed from soluble impurities, the desired functionalized hydrogels were recovered from the solution by freeze drying.

### Example 7. Composition comprising boron compounds, cell adhesion peptides domains and a support comprising a functionalised surface, for instance with TEG linkers.

For platforms presenting cell adhesion peptide domains, it has been followed maleimide-thiol coupling strategy. Hydroxyl groups of the TEG-functionalised substrates are reacted with N-Maleoyl-β-glycine and the appropriate cysteine-terminated commercial peptides containing specific integrin adhesion sequences (SEQ ID NO: 3 - GRGDSPC- from FN and SEQ ID NO: 4 - CSRARKQAASIKVAVSADR - from LN). This is an established coupling process that occurs spontaneously between the -SH groups included on the cysteine aminoacids and the maleimide-functionalized TEG. In addition, due to the absence of activation reagents and reaction byproducts, together with the possibility of carrying out the reaction in aqueous media, makes this strategy highly robust for anchoring any Cys-containing peptide (see scheme 3).

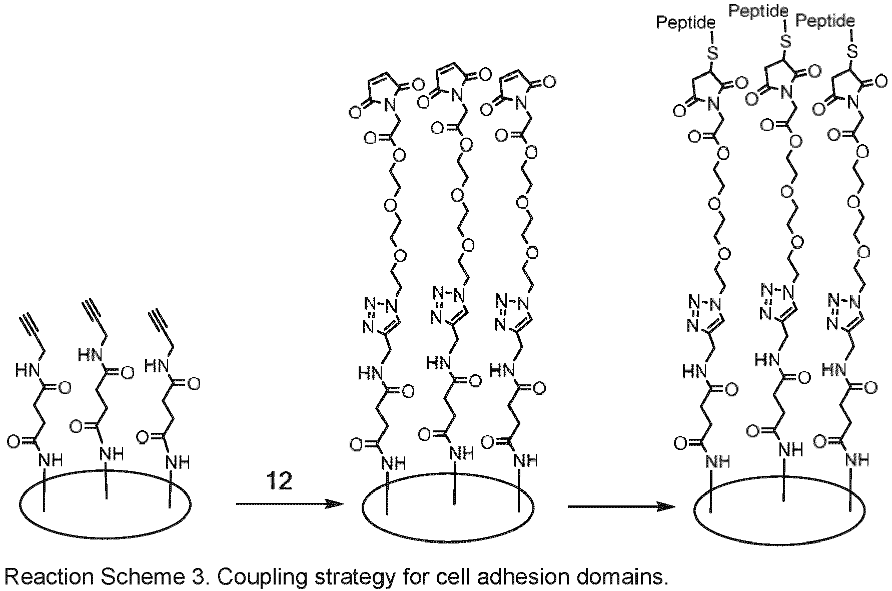

For compositions comprising both Boron compounds and cell adhesion peptide domains, the reaction performed was a stoichiometric reaction of both, azide-containing TEGs and silanized substrates. This strategy provides a surface with two-different bioactive fragments (see scheme 4 which also allows further modifications of such moieties following the procedures explained heretofore.

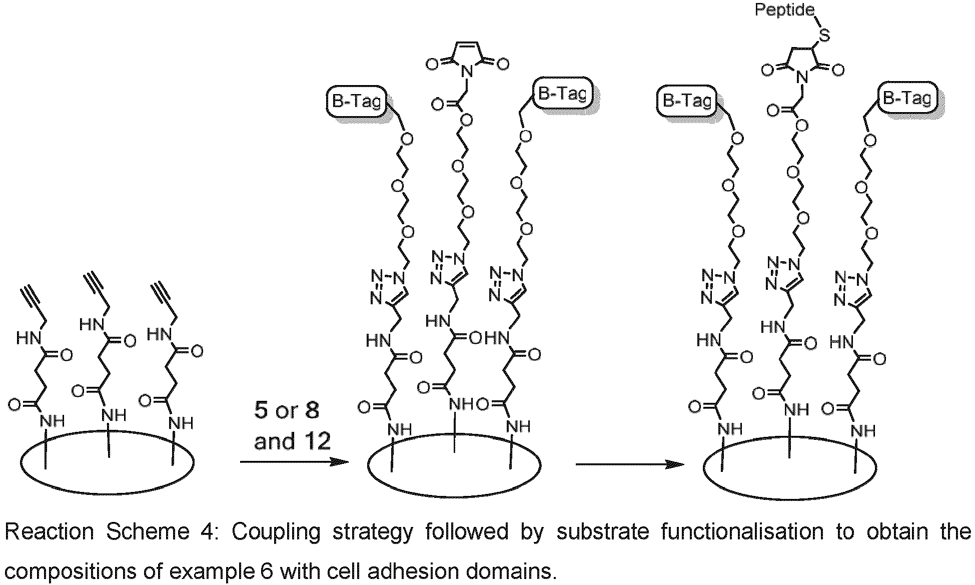

### Example 8. Composition comprising boron compounds, cell adhesion peptides domains and a hydrogel support comprising a functionalised surface with TEG linkers

Polyethylene glycol (PEG) has a well stablished chemistry and long history of safety in *in vivo* experimentation. In addition, PEG structure is highly modifiable and almost every aspect can be altered to optimise the system and its properties, such as incorporation of biomolecules, protease-degradable sites, adhesive ligands and GFs. The PEG hydrogels used were formulated using 4-armed polyethylene glycol with maleimide moieties at each terminal chain (PEG-4MAL). The branched form is used to form hydrogels after terminal chain functionalisation. Customised cysteine terminated commercial peptides containing the cell adhesion sequences for different integrin stimulation, SEQ ID NO: 3 - GRGDSPC (containing SEQ ID NO: 1 - RGD - from Fibronectin-FN) or SEQ ID NO: 4 -CSRARKQAASIKVAVSADR - (containing SEQ ID NO: 2 - IKVAV - from Laminin-LN) peptides were obtained and bound to a small proportion of the maleimide groups (PEGylation) through a Michael-type addition reaction at pH 7 between the maleimide and the thiol groups present in the cysteine amino acids.
PEG-4MAL molecules were covalently bound together by the same mechanism using another customised commercial peptide, VPM (SEQ ID NO: 5 - GCRDVPMS↓MRGGDRCG), flanked by two cysteine residues as a cross-linking. The cross-linking peptide also contained a sequence cleavable by matrix metalloproteases (cleavable site indicated as ↓), allowing its degradation.

Once the PEG-based hydrogel support was optimised in terms of composition and degradability, PEG-4MAL molecules were covalently bonded to commercial thiol containing boronic acid, or other suitable boron compounds, for generation of the B-covalently bound systems, for simultaneous NaBC1- integrin stimulation.

### Example 9. Composition comprising boron compounds, cell adhesion peptides domains and an alginate-based hydrogel support comprising a functionalised surface with TEG linkers.

Alginate based hydrogels also have a well stablished chemistry and are of increasing interest in therapeutic applications or as a matrix for 3D cell culture. In a first preparation stage, ultrapure sodium alginate is dissolved in 1% D-mannitol at a concentration of 1.5% and filtered through a 0.22 µm pore filter for sterilisation. For gelation, 1.5% alginate was mixed with CaSO₄·2H₂O through two LuerLock syringe connected with a Fluid Dispensing connector until complete homogenisation. Alginate hydrogels were customised with commercial peptides containing the cell adhesion sequences for different integrin stimulation. RGD domain from fibronectin (FN) or the IKVAV domain from Laminin (LN) peptides were bound to a small proportion of the alginate chains through an amidation reaction (scheme 5 and 6).

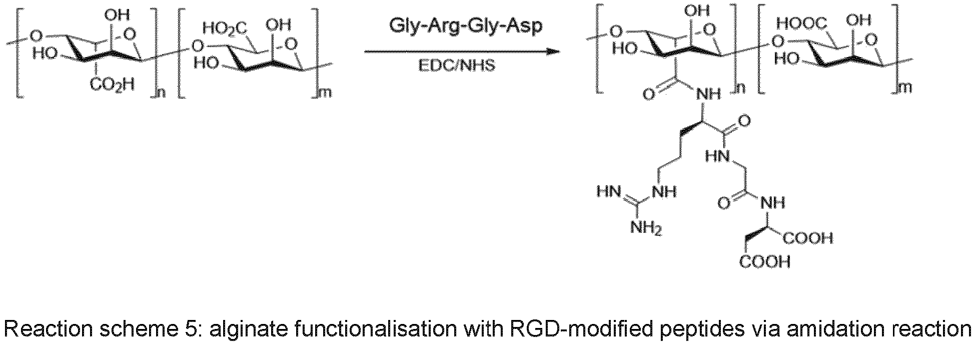

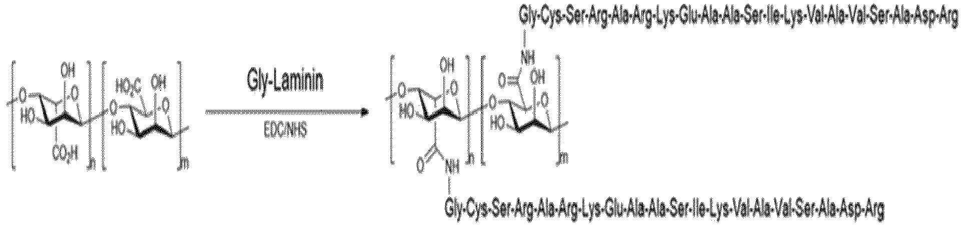

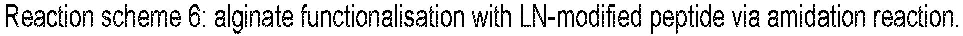

Prior peptide functionalisation of hydrogels, SEQ ID NO:1 - RGD - and SEQ ID NO: 2 - IKVAV - peptides were modified by addition of Glycine in order to provide more reactive NH₂ groups to further amidation reactions (**reaction scheme 7**).

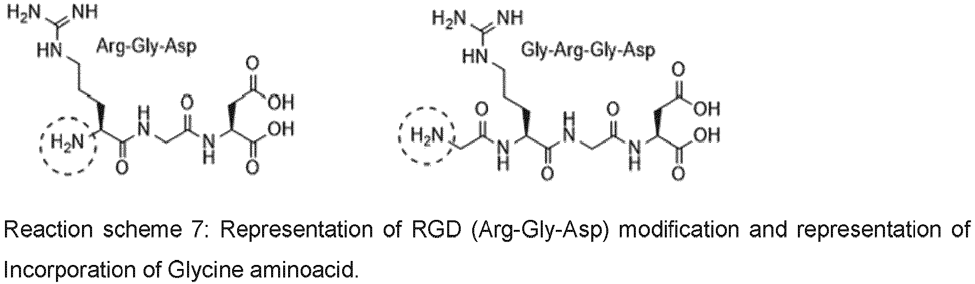

Incorporation of Glycine aminoacid provides a more accessible anchoring point to favour amidation reaction for alginate functionalisation. Once the alginate-based hydrogel system was optimised in terms of composition with cell adhesion domains, alginate molecules were covalently bonded to commercial thiol containing boronic acid (or any of the other boron compounds of the invention) for generation of the B-covalently bound systems, for simultaneous NaBC1-integrin stimulation. Bis(pinacolato)diboron and bortezomib can also be used as the boron compound generation of the B-covalently bound systems.

### Example 10: General Procedure for the synthesis of mesoporous silica nanoparticles and the preparation of mesoporous silica nanoparticles supports having a functionalized surface with TEG linkers suitable to provide composition comprising a boron compound, and adjuvant (fibronectin or cell adhesion domains) and a hydrogel support comprising TEG as linkers.

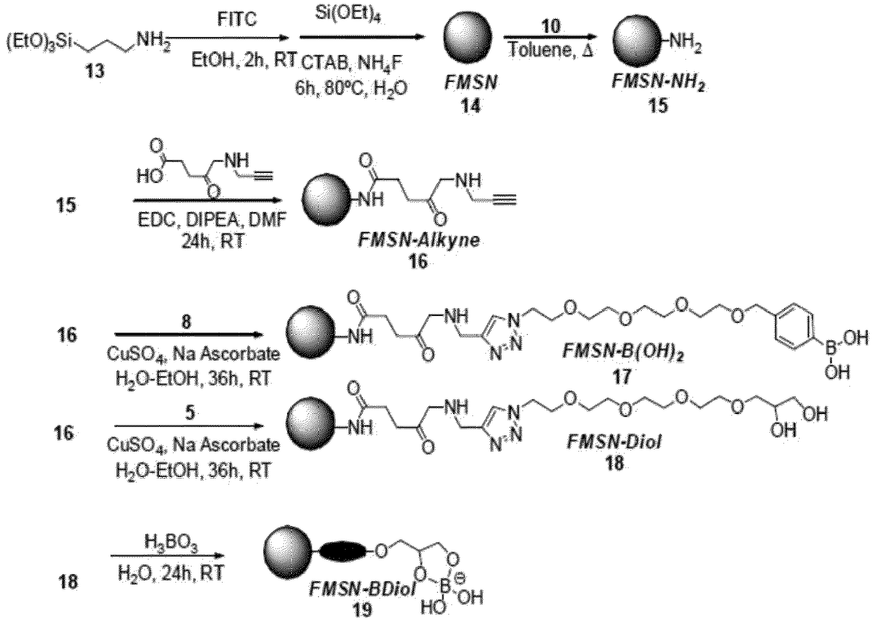

### Synthesis of fluorescent mesoporous silica nanoparticles (FMSN, 14) supports.

Fluorescent-tagged MSNs were synthesized according to a previously reported procedure. Briefly, in a 1 L round bottom flask was prepared a solution of 1.82 g CTAB (5 mM) and 3 g NH₄F (81 mM) in 500 mL of water; the solution was heated to 80° C for at least 30 minutes. Meanwhile, in a vial were reacted fluorescein isothiocyanate (FITC, 3mg, 7.7 µmol) with 3-(aminopropyl)triethoxysilane (APTES, **13** 1.5µL, 6.5 µmol) in 100 µL EtOH at RT for 30 minutes. When completed, the reaction is diluted with TEOS (9 mL, 8.41 g) and the result is slowly added over the CTAB solution under vigorous stirring. The reaction is kept at 80° C for 6 h and the prepared nanoparticles are collected by centrifugation (12.000 rpm, 10 min). The prepared *FMSNs* are washed with water and ethanol three times and dried at 40° C under vacuum. Prepared *FMSNs* showed a hydrodynamic diameter measured in deionized water by Dynamic Light Scattering (DLS) of 133.7 ± 2.53nm and a ζ-potential of +14.7 ± 1.31 mV.

### Silanization of fluorescent mesoporous silica nanoparticles with 3-(aminopropyl)-triethoxysilane (FMSN-NH2, 15).

To modify the *FMSN's* surface, the obtained dry particles were dispersed in 750 mL toluene containing 500 µL APTES in a 1 L round bottom flask fitted with a Dean-Stark and a reflux condenser. The system was condensed at reflux during 20 h. When finished, the particles were centrifuged and washed with EtOH twice. Then, to remove the surfactant (CTAB), the product was treated with two 24 h cycles of reflux (80° C) employing acidic EtOH (400 mL of ethanol containing 8 mL of concentrated HCI). Finally, the prepared *FMSN-NH₂* were washed three times with EtOH and dried under vacuum. The resulting *FMSN-NH₂* particles showed a DLS hydrodynamic diameter of 176.2 ± 3.79 nm and a ζ-potential of +27.0 ± 0.48 mV. Quantification of amino groups was achieved by two-step protection and cleavage with Fmoc-CI (λmax=301nm), determining a modification of about 0.42 mmol NH₂/g.

***Coupling of FMSN-NH2 with 4-oxo-5-(prop-2-yn-1-ylamino)pentanoic acid (FMSN-Alkyne,* 16).** For the coupling of the amino-capped *FMSN-NH₂* particles with the alkyne containing moiety was employed a known amide-bond formation protocol. Briefly a suspension of *FMSN-NH₂* (250 mg) in DMF (5 mL) was reacted with a solution of 9 (50mg, 0.32 mmol), EDC (93 mg, 0.48 mmol), DIPEA (225µL, 1.30 mmol) and NHS (37 mg, 0.32 mmol) in dry DMF (5 mL) for 24 h at RT. Then, the particles were centrifuged and washed with DMF (2 x 20mL) and EtOH (3 x 20mL) and dried under vacuum. The resulting particles showed a DLS hydrodynamic diameter of 176.2 ± 3.79 nm and a ζ-potential of +27.0 ± 0.48 mV.

**Coupling amino-modified nanoparticles with boron-containing azide-capped TEG. (FMSN-B(OH₂), 17 and FMSN-Diol, 18):** Cu-catalyzed click chemistry coupling of nanoparticles was accomplished employing the previously prepared particles 16 with azide-containing compounds 5 or 8. Briefly, over a suspension of 16 (100 mg) in EtOH:H2O (1:1, 10 mL) was added a solution of CuSO4·5H2O (4.8 mg, 30 µmol), sodium ascorbate (595 mg, 3 mmol) and either 5 (88 mg, 0.3 mmol) or 8 (106 mg, 0.3 mmol) in EtOH:H2O (1:1, 20 mL). The reaction was stirred at RT for 36 h and then centrifuged and washed twice with EtOH:H2O (1:1, 40 mL) and EtOH (2 x 50mL) and finally dried under vacuum. The resulting particles showed a DLS hydrodynamic diameter in water of 148.2 ± 6.22 nm and a ζ-potential of 13.80 ± 0.92 mV for 17 (TEM images) and 141.2 ± 1.95 nm and a ζ-potential of +11.1 ± 0.74 mV for 18.

### Example 11. Compositions comprising engineered supports of nanoparticles for site-directed activation of NaBC1 functionalised with TEG linkers and cell adhesion peptide domains (Compounds 17 and 18)

A similar technology explained for aforementioned material systems was employed for functionalisation of diverse types of nanoparticles. The nanoparticles display B or/and cell adhesion domains in their corona after covalent binding with TEG chains, stimulating specifically NaBC1 and integrins. These nanoparticle platforms are potentially very useful for medical applications that require parenteral administration and allow specific delivery at the nanoscale.

### Example 12: Preparation of topical compositions comprising 4 bis(pinacolato)diboron, cell adhesion domain peptide SEQ ID NO: 3 - GRGDSPC and a hydrogel support comprising TEG as linkers

The composition of example 7 was prepared as described in said example. Afterwards, said composition was mixed with water for injection (WFI), triethanolamine and citric acid under high shear conditions (paddle mixer and sonication) at a temperature from 40 to 50 degrees Celsius. Afterwards glycerol was added under the aforementioned conditions. See table 3.

### Example 13: Preparation of topical compositions comprising bortezomib cell adhesion domain peptide SEQ ID NO: 1- RGD and a hydrogel support comprising TEG as linkers

The composition of example 7 was prepared as described in said example using bortezomib as the boron compound and the SEQ ID NO: 1- RGD as the cell adhesion peptide domain. Then, said composition was mixed with water for injection (WFI), triethanolamine and citric acid under high shear conditions (paddle mixer and sonication) at a temperature from 40 to 50 degrees Celsius. Afterwards glycerol was added under the aforementioned conditions. See table 3.

**Table 3: topical compositions comprising bis(pinacolato)diboron or bortezomib**

| Ingredients | Amount | %W | Amount | %W |
|---|---|---|---|---|
| | Composition 7 | | Composition 8 | |
| bis(pinacolato)diboron | 76.53 µg | 1.91 | NA | NA |
| Bortezomib | NA | | 1.1 10⁻⁴ µg | 2.8 10⁻⁶ |
| Peptide domain SEQ ID NO: 1 | NA | | 69 µg | 1.73 |
| Peptide domain SEQ ID NO: 1 | 69 µg | 1.73 | NA | NA |
| Support | 0.33-1.39 mg | 8.36-34.86 | 0.41-1.47 mg | 10.27-36.77 |
| Glycerol | 0.02-0.16 mg | 0.5-4 | 0.02-0.16 mg | 0.5-4 |
| Citric acid | 0.04-0.16 mg | 1-4 | 0.04-0.16 mg | 1-4 |
| Water | 2.4-3.2 mg | 60-80 | 2.4-3.2 mg | 60-80 |

## Claims

1. Combination of a boron compound and an adjuvant for use in a method to induce myotube formation, increase myotube diameter and/or suppressed cell mortality in a mammal in need thereof;
wherein the adjuvant comprises fibronectin or cell adhesion peptide domains, provided that when the adjuvant is fibronectin, the boron compound is not borax;
wherein when the adjuvant comprises fibronectin, the ratio of boron compound to the adjuvant is from 1.10⁻¹ to 1.10⁻⁶ by weight and wherein the adjuvant comprises cell adhesion peptide domains, the ratio of boron compound to the adjuvant is from 10 to 1.10⁻⁶ by weight; and wherein the cell adhesion peptide domains comprise a sequence selected from a list consisting of the SEQ ID NO: 1, EQ ID NO: 2, SEQ ID NO:3 and SEQ ID NO: 4.

2. The combination according to any of the previous claims, wherein when the boron compound is bortezomib, the ratio of bortezomib to the cell adhesion peptide domains is from 5 to 1.10⁻⁶ by weight.

3. A pharmaceutical composition comprising;
- A boron compound;
- at least an adjuvant which comprises fibronectin or cell adhesion peptide domains, provided that when the adjuvant is fibronectin, the boron compound is not borax; and
- wherein when the adjuvant comprises fibronectin, the ratio of boron compound to the adjuvant is from 1.10⁻¹ to 1.10⁻⁶ by weight and/or wherein when the adjuvant comprises cell adhesion peptide domains, the ratio of boron compound to the adjuvant is from 10 to 10⁻⁶, preferably from 5 to 10⁻⁶;
- Optionally at least pharmaceutical acceptable excipient; and
wherein the cell adhesion peptide domain is a peptide which comprises or consists of a sequence selected from a list consisting of the SEQ ID NO: 1, SEQ ID NO: 2, SEQ ID NO: 3 and SEQ ID NO: 4.

4. The composition according to claim 3, wherein when the boron compound is bortezomib and the ratio of bortezomib to the cell adhesion domain is selected from 1.10⁻¹ to 1.10⁻⁶.

5. A pharmaceutical composition comprising;
- A boron compound;
- at least an adjuvant which comprises fibronectin or cell adhesion peptide domains; wherein the cell adhesion peptide domain is a peptide which comprises or consists of a sequence selected from a list consisting of the SEQ ID NO: 1, SEQ ID NO: 2, SEQ ID NO: 3 and SEQ ID NO: 4;
- a support which comprises a substrate selected from the list consisting of hydrogel matrices, glassware and silica nanoparticles; and
- optionally a pharmaceutically acceptable excipient.

6. The pharmaceutical composition according to the previous claim, wherein the support comprises a surface functionalized with a linker, wherein the linker is bonded to at least one boron compound.

7. The pharmaceutical composition according to the previous claim, wherein the linker is selected from the list consisting of tetraethyleneglycol, pentaethyleneglycol, hexaethyleneglycol, heptaethyleneglycol, octaethyleneglycol, PEG having a molecular weight Mn from 200 to 2000, measured by GPC; preferably the linker is tetraethyleneglycol.

8. The pharmaceutical composition according to any of the claims 5-7, wherein the boron compound is selected from, 2-boronoethyl, 3-boronopropyl, m- and p boronophenyl and pinacolato thereof, 1-Propylboronic acid catechol ester, Ethyl dihydrogen borate, triethylene borate, 2-Aminoethyl diphenylborinate, 2-hydroxy-1,3,2-dioxaborolan-4-yl)methoxyl, 4-Hydroxy-3-methoxyphenylboronic acid pinacol ester, 2-((2- hydroxy benzo[d][1,3,2]dioxaborol-5-yl)oxyl, m-(boronooxy)phenoxyl, p-(boronooxy)phenoxyl, bortezomib, tavaborole and crisaborole.

9. The pharmaceutical composition according to any of the claims 5-8, wherein the surface of the support is further bonded to the adjuvant through the linker, preferably is further bonded to through the same or through a different linker and /or wherein the linker is bonded to at least one boron-containing compound.

10. The pharmaceutical composition according to the previous claim, wherein the bond between the boron compound and the linker is a hydrolysable bond or a covalent bond.

11. The pharmaceutical composition according to any of the claims 3-4 or the pharmaceutical composition according to any of the claims 5-10, wherein the composition is an injectable composition, preferably an injectable subcutaneous or intramuscular composition or wherein the composition is an oral composition in the form of a tablet, capsule, cream, gel, liquid or a powder or a topical composition in the form of a cream, ointment, gel, liquid or a dispersible powder.

12. The combination according to any of the claims 1-2 or the pharmaceutical compositions according to claims 3-11, for use in a method to improve muscle regeneration or for use in a method of treatment or therapy of pathophysiological condition which affects skeletal muscle, preferably said condition is a dystrophy selected from the list consisting of myotonic dystrophia type 1, myotonic dystrophia type 2 and Duchenne muscular dystrophia.

13. The combination according to any of the claims 1-2 or the pharmaceutical compositions according to claims 3-11, for use in a method of treatment or therapy of pathophysiological condition which affects skeletal muscle, wherein said method comprises administering the boron compound to said mammal in an amount effective for decreasing muscle atrophy and/or in amount effective to increase muscle growth in said mammal, preferably wherein the boron compound is bortezomib and is administered to a human in an effective dose from 0.1 to 1 mg once or twice weekly, preferably, from 0.1 to 0.5 mg once or twice weekly.

14. The combination according to any of the 1-2 or the pharmaceutical compositions according to claims 3-11, for use as regenerative therapeutic treatments selected form the list consisting of, muscle repair, muscle regeneration, as a consequence of an injury due to sport lesions, injuries in general or muscle defects naturally generated by aging.

15. A method to prepare the composition according to any of the claims 5-11, which comprises at least the steps of:
a) Preparing the functionalised support by chemically modifying the surface of the support by silanization and further reacting with a compound having a terminal alkyne group;
b) Incorporating the boron compound in the functionalised support of the previous step by performing a coupling reaction on the product obtained in the previous step using a Pd-mediated cross-coupling agent to incorporate the boron compound into the linker, wherein the linker chain comprises a terminal azide group;
c) azide-alkyne click coupling between the modified surface from step (a) and the boron-containing linker from step (b);
d) performing a maleimide-thiol coupling between the support obtained in step (c) and adjuvant, wherein the adjuvant contains the appropriate cysteine-terminated peptides containing specific integrin adhesion sequences, preferably wherein the adjuvant is Fibronectin or a Cell adhesion domain peptide, which comprises or consists of a sequence selected from a list consisting of the SEQ ID NO: 1, SEQ ID NO: 2, SEQ ID NO: 3 and SEQ ID NO: 4;
